(19) Europäisches Patentamt
European Patent Office
Office européen des brevets

(11) EP 4 089 133 A1

(12) **EUROPEAN PATENT APPLICATION**

(43) Date of publication:
**16.11.2022 Bulletin 2022/46**

(21) Application number: **21173944.6**

(22) Date of filing: **14.05.2021**

(51) International Patent Classification (IPC):
***C08G 65/22*** *(2006.01)* ***A61K 47/60*** *(2017.01)*
***C08G 65/333*** *(2006.01)*

(52) Cooperative Patent Classification (CPC):
**C08G 65/22; A61K 47/60; C08G 65/33331; C08G 65/33396**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(71) Applicant: **Johannes Gutenberg-Universität Mainz 55122 Mainz (DE)**

(72) Inventors:
- **Frey, Holger**
  **79312 Emmendingen (DE)**
- **Mohr, Rebecca**
  **55116 Mainz (DE)**
- **Dreier, Philip**
  **55122 Mainz (DE)**

(74) Representative: **Durm Patentanwälte PartG mbB**
**Patentanwälte**
**Moltkestrasse 45**
**76133 Karlsruhe (DE)**

# (54) POLY(ETHYLENE GLYCOL) HAVING C1 TO C3-ALKYLOXYMETHYL SIDE CHAINS, BIOCONJUGATES THEREOF, PROCESS FOR ITS PREPARATION AND ITS USE.

(57) The present invention concerns polyether polymers represented by the following formula [I]:

$$-(CH_2CHR\text{-}O\text{-})_m- \quad [I],$$

wherein residues R are hydrogen or C1-C3 alkoxymethyl, 1 to 100 % are methoxymethyl and up to 50% of R may be C2-C3 alkoxymethyl with the proviso that at least one R is hydrogen, if at least one R is C2-C3 alkoxymethyl; and m is in the range of from 10 to 1000, wherein the dispersity is 1.15 or less. The invention further concerns a process for their preparation, conjugates thereof and the use thereof.

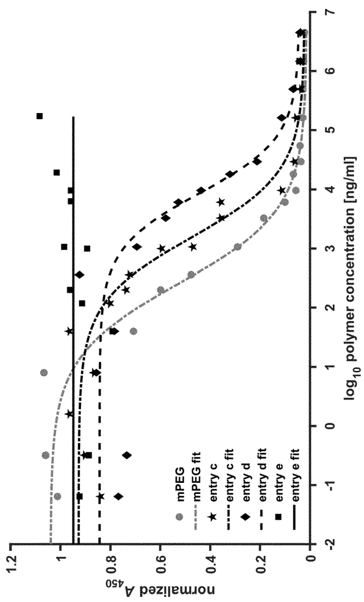


Figure 1

EP 4 089 133 A1

## Description

### Introduction

The present invention concerns polyether polymers represented by the following formula [I]:

$$-(CH_2CHR\text{-}O\text{-})_m\text{-} \quad [I],$$

wherein residues R are hydrogen or C1-C3 alkoxymethyl, 1 to 100 % are methoxymethyl and up to 50% of R may be C2-C3 alkoxymethyl with the proviso that at least one R is hydrogen, if at least one R is C2-C3 alkoxymethyl; and m is in the range of from 10 to 1000, wherein the dispersity is 1.15 or less. The invention further concerns a process for their preparation, conjugates thereof and the use thereof.

### Background of the invention

The aliphatic polyether poly(ethylene glycol) (PEG) is the most widely employed polymer in pharmaceutics, medicine and drug delivery. PEGylation, i.e. the attachment of PEG to peptide drugs, was introduced in the 1970ies and has since then led to numerous highly efficient drugs. PEGylation is a form of conjugation, i.e. the attachment of a polymer to a molecule by a covalent bond. The main effect of the attachment of PEG chains is the so-called "stealth effect", which prevents close approach of and recognition by the immune system and subsequent binding and removal by the reticuloendothelial system (RES) in the liver. More simply, PEG attachment also prevents opsonization by proteins, when attached to the surfaces of nanocarriers for drug delivery. The inhibition of protein adsorption by PEG is further emphasized by fulfillment of the rules for protein-repellent surfaces derived by Whitesides (Yarovsky et al., "Quantitative design rules for protein-resistant surface coatings using machine learning", Scientific Rep. 2019, 9, 265). PEGylation is also used for "stealth liposomes" with long circulation times, low molecular weight drugs as well as for lipid nanoparticles. Such structures are employed for the transport and delivery of mRNA. Here the respective PEG-based lipids play a key role for the vaccination against the COVID-19 pandemic, enabling transport of mRNA to cells, avoiding undesired degradation processes. The recently approved mRNA vaccines from Moderna and Pfizer-BioNTech require PEGylated lipids to ensure the stability of the mRNA in the lipid-based nanoparticle. PEGylation also prevents coalescence of liposomes and similar structures in aqueous systems. Commonly, molecular weights in the range of 2 to 40 kg/mol are preferred for the PEGylation of drugs or nanocarriers. To sum up, PEG has become a standard for watersoluble medical polymers for a vast variety of medical and pharmaceutical uses, particularly in the therapy of cancer, chronic diseases and recently also for RNA delivery vehicles. The success of PEG may have had several reasons. Among them are commercial availability in sufficient quality and in a wide range of molar masses from the anionic ring-opening polymerization of ethylene oxide as well as its extremely low toxicity and high biocompatibility. Further, PEG can be prepared in narrow molecular weight distributions.

However, an increasing number of studies summarize concerns related to the presence of so-called anti-PEG antibodies (APAs) that are present in a large and constantly growing part of the population. Such antibodies can lead to accelerated clearance of PEGylated drugs from the blood stream, i.e. loss of the stealth effect that is crucial for therapeutic success, allergic reactions and in extreme and rare cases even potentially fatal anaphylactic shock. This undesired effect is observed both for PEGylated peptide drugs, PEGylated liposomes and other PEGylated nanocarriers as well as PEGylated small molecule drugs. Anti-PEG antibodies are also problematic with respect to PEGylated lipids for RNA-transporting lipid nanoparticles, e.g. in the context of COVID-19 vaccines. PEG-antibodies are often mentioned in the context of potential PEG-alternatives as a motivation to look beyond PEG for alternatives. As an estimate, approximately 10% of the population experience allergic reactions when treated with PEGylated formulations. Only recently, the nature of the binding between PEG and anti-PEG antibodies has been studied by Lai et al (Lai et al, "Structure of an anti-PEG antibody reveals an open ring that captures highly flexible PEG polymers." Commun Chem 2020, 3, 124). They concluded that binding of PEG is due to an open ring structure of PEG captured by the APAs. By counting the number of monomer repeats of the PEG polymer interacting with the interior and exterior paratope of the Fab, they found the size of the PEG antigen epitope to be ~700 g/mol, equivalent to 16 monomer subunits. Thus, at least 16 ethylene glycol units are required for binding by the APA. Other works suggest that also shorter regular chain segments of PEG as well as the methoxy end group of mPEG ($\alpha$-methoxy poly(ethylene glycol)) structure play a key role for the recognition. US 8,129,330 B2 therefore discloses methods for the preparation of PEG conjugates that are based on end group modification of the PEG chains. Variation of the end group of PEG is disclosed, aiming at minimizing interaction of for example methoxy end groups with anti-PEG antibodies. The authors disclose conjugates, wherein a hydroxyl group is present on all of the distal polyalkylene glycol termini in said pure conjugate, and wherein said conjugate exhibits reduced antigenicity compared to mPEG-protein conjugates. It has been reported that antibodies elicited by PEG-OH have similar affinity to both mPEG and PEG-OH, while antibodies induced by mPEG recognize mPEG more effectively than PEG-OH (Sherman et al, "Role of the methoxy group in immune responses to mPEG-protein conjugates." Bioconjug Chem 2012, 23(3), 485-499; Sherman et al, Selectivity of binding of PEGs and PEG-like oligomers to anti-PEG antibodies induced by methoxyPEG-proteins." Mol Immunol 2014, 57(2), 236-246.). These results have been obtained by using competitive ELISA and imply that the anti-PEG antibodies elicited by PEG-OH-proteins are directed against the backbone of the PEG (backbone-specific), while antibodies induced by mPEG-protein conjugates are methoxy group specific. It can be concluded that regular segments of at least 4-5 to 16 regularly arranged ethylene glycol units are required to achieve recognition and immunogenic reaction by anti-PEG antibodies. Recent clinical works demonstrated that the presence of antibodies against PEG permits to predict Pegasparagase allergic reactions and failure of rechallenge, emphasizing the clinical importance of anti-PEG antibodies for the success of the treatment of leukemia in this case (Liu et al, "Antibodies Predict Pegasparagase Allergic Reactions and Failure of Rechallenge", J. Clin. Oncol. 2019, 37, 2051).

Three strategies for the modification of the PEG structure have been developed to counteract undesired APA interaction of PEGylated therapeutics. Replacing the methoxy end group of mPEG by a hydroxyl group led to a strong decrease in the affinity for anti-PEG antibodies, which was demonstrated by competitive ELISA tests. However, it is obvious that for large scale commercial applications the presence of a hydroxyl group at the terminus would require protected functional initiators, which complicates existing synthetic protocols. A second recent strategy is disclosed in US 2019/0015520 A1 and relies on PEG-bottlebrush structures, i.e. small PEG structures appended to a poly(hydroxyethyl methacrylate) backbone. The inventors state that this type of PEG-architecture minimizes anti-PEG antigenicity, while preserving the desired stealth properties for surface coating. However, the resulting PEG-grafted copolymers exhibit rather broad, multimodal molecular weight distributions, which represent a major obstacle for approval for medical application and PEGylation analogous bioconjugation with peptides or lipids. Narrow distributions (Mw/Mn < 1.15) were exclusively feasible by performing a cost- and time-consuming preparative size-exclusion chromatography (SEC).

Another strategy is to exchange PEG with the linear polyether polyglycerol to increase the blood circulation time of liposomes and to avoid accelerated blood clearance (see Abu Lila et al, "Use of polyglycerol (PG), instead of polyethylene glycol (PEG), prevents induction of the accelerated blood clearance phenomenon against long-circulating liposomes upon repeated administration", International Journal of Pharmaceutics, 456 (2013) 235-242). However, the utilization of polyglycerol also results in the introduction of multiple hydroxyl functionalities to the liposome, which is in contrast to the aforementioned rules of Whitesides resulting in complex unspecific recognitions of peptides in the blood stream. Therefore, a targeted use of the conjugated drug or liposomes is not possible with polyglycerol.

Since PEGylated therapeutics are often used for chronic or in many cases otherwise fatal diseases, like in cancer therapy, in many cases repeated doses have to be administered over prolonged periods of time. PEG is a non-biodegradable polymer and has to be eliminated from the body through the kidneys. In studies on the renal filtration of PEGylated drugs, an undesirable accumulation of PEG in the kidneys and the associated pathological changes in the renal system could be demonstrated (see e.g. Bendele, A.; Seely, J.; Richey, C.; Sennello, G.; Shopp, G. Short Communication: Renal Tubular Vacuolation in Animals Treated with Polyethylene-Glycol-Conjugated Proteins. Toxicol. Sei. 1998, 42, 152-157). The tendency of polyethylene to crystallize can therefore lead to deposits in the kidney. The elimination through the kidneys also limits the molecular weight of PEG for medical therapeutics to the renal cutoff size, which for globular proteins is approximately 50 to 60 kDa.

The present inventors have reported in 2014 on the copolymerization of ethylene oxide with glycidyl methyl ether (GME) by use of the monomer activation method and tetraoctylammonium bromide and tris-*iso*-butylaluminum as initiators (see Frey et al, A Challenging Comonomer Pair: "Copolymerization of Ethylene Oxide and Glycidyl Methyl Ether to Thermoresponsive Polyethers", Macromolecules 2014, 47, 5492-5500). Rather broad molecular weight distributions were obtained (dispersity in the range of 1.21 to 1.5), which are clearly not suitable for medical applications. As discussed at the time, with the conventional anionic ring-opening method only molecular weights up to 3000 g/mol and high dispersities have been reported for the polymerization of GME. In previous studies regarding the homopolymerization of GME to poly(glycidyl methyl ether) (PGME) it was generally stated that polymerization causes difficulties, and no molecular weight control can be achieved by anionic ring-opening polymerization as employed for medical PEG (Frey et al., Biomacromolecules 2014, 15, 1935-1954). In all available reports regarding GME polymerization to date, either phosphazene bases were used as a catalyst or aluminum compounds had to be added in the way of a "monomer activated polymerization". None of these polymers or copolymers are suitable for medical applications for several reasons: (i) traces of phosphazene bases lead to toxicity and cannot be removed; (ii) dispersities exceed 1.1, which is prohibitive for most medical applications and (iii) end-group fidelity of the polymers derived from the methods is insufficient for attachment to peptides, drugs or lipids via bioconjugation. Therefore, neither PGME homopolymer nor copolymers of GME have been considered for PEGylation type applications to date.

The retention of polymer end-groups (end-group fidelity) is a critical tool for chain extension and post polymerization reactions. End-group fidelity is also a decisive factor for the use of polymers in bioconjugates for pharmaceutical applications, in which the biomolecules are connected by means of the end-groups. Polymers with low end-group fidelity comprise a large amount of macromolecules that are not bound to the biomolecule and cannot be removed from the mixture without undue expenditure. The formed conjugates do not have the required purity. The loss of end-groups is

therefore not desirable, but cannot be completely avoided. End-group fidelity varies greatly depending on the reaction conditions in the preparation of the polymers.

Purpose of the invention

It is the objective of the present invention to provide alternative polymers that are able to replace PEG in its applications and that can alleviate at least some of the aforementioned disadvantages of PEG. The alternative polymer should especially be suited to replace PEG in pharmaceutical applications. It is further an objective to provide polymers with low dispersity. A further objective is to provide polymers with high end-group fidelity. It is also desired to provide a process that allows preparation of such polymers in a simple and commercially feasible way. The alternative polymers should have a low affinity towards anti-PEG antibodies (APAs). A further goal is to provide polymers with a low immunogenicity.

Detailed description

The present invention concerns poly(ethylene glycol) having C1 to C3-alkyloxymethyl side chains. The present invention concerns polyether polymers represented by the following formula [I]:

$$-(CH_2CHR\text{-}O\text{-})_m- \qquad [I]$$

wherein residues R are independently from each other selected from the group consisting of hydrogen, methoxymethyl, ethoxymethyl, n-propoxymethyl and iso-propoxymethyl; 1 to 100% of the residues R are methoxymethyl; up to 50% of the residues R may be selected from the group consisting of ethoxymethyl, n-propoxymethyl and iso-propoxymethyl; with the proviso that at least one residue R is hydrogen, if at least one residue R is selected from the group consisting of ethoxymethyl, n-propoxymethyl and iso-propoxymethyl; and m is in the range of from 10 to 1000, characterized in that the dispersity is 1.15 or less. Herein the percentages of the residues R are based on the sum of all residues R, i.e. on m. These new polyether polymers are a new type of material, which behaves very different to PEG in regard to the affinity towards anti-PEG antibodies (APAs) and have a low immunogenicity. They also have a low crystallinity. But the polyether polymers of the present invention behave very similar to PEG with respect to various features that are important for pharmaceutical applications, including aqueous solubility, hydration, cell viability and biocompatibility.

Key to the reduced interaction with APAs is the use of substituents on the PEG backbone. The increased spatial requirements of the said PEG copolymers impede or disable interaction with anti-PEG antibodies according to the specific "lock and key principle". Besides the steric impact, the random distribution of the substituents over the polymer backbone and/or the random steric orientation of the substituents furthermore disable the development and formation of specific anti-polymer antibodies.

Key to the similarity of the features of PEG and the inventive polymers is the use of alkoxymethyl side chains. This maintains water solubility and other properties in view of PEG. A repeating group of the present polymer wherein R is a methoxymethyl group, i.e. $(-CH_2-CH(-CH_2-OCH_3)-O)-$, has the same molecular formula $C_4H_8O_2$ as two repeating units of PEG $(-CH_2-CH_2-O)_2-$ and is therefore a constitutional isomer of two main chain repeating units of PEG. It comprises the same number of atoms and very similar functional groups. This leads to similar properties, especially to similar water solubility, which is important for applications in biological systems. The lack of reactive functional groups in the side chain like hydroxy, amino etc. reduces undesired interactions with biological systems.

Ethoxymethyl, n-propoxymethyl and iso-propoxymethyl may be used as residue R to adjust the properties of the polymers further, for example their solubility, interaction with lipids, properties in respect to the passage of biological membranes etc. However, these residues lead to a reduced solubility in water. The choice of such substituents also leads to a deviation from the behavior of PEG for other properties. For this reason, the amount of repeating units with the residues selected from the group consisting of ethoxymethyl, n-propoxymethyl and iso-propoxymethyl is limited to 50% of all residues R of the polymer of formula (I). Also in embodiments of the present invention in which at least one residue R of the polymers is selected from the group consisting of ethoxymethyl, n-propoxymethyl and iso-propoxymethyl at least one residue R must be hydrogen, preferred at least 5 % are hydrogen in such embodiments, more preferred at least 10 % are hydrogen and most preferred at least 25 % are hydrogen. In a further preferred embodiment up to 20% of the residues R may be selected from the group consisting of ethoxymethyl, n-propoxymethyl and iso-propoxymethyl and even more preferred only up to 5% of the residues R may be selected from this group. These residues are not required for some of the uses of the polymers of the present invention in pharmaceutical applications. For this reason, polymers of the present invention are preferred where R is selected from the group consisting of hydrogen and methoxymethyl. Obviously, ethylene oxide repeating units in the inventive polymers also lead to similar properties with PEG. This selection therefore provides the polymers that are closest in behavior to PEG.

Higher homologues of methoxymethyl, i.e. where R is C4 alkoxymethyl and higher lead to increasingly low

solubility in water and other detrimental properties and should therefore generally be avoided. They may however be used in small quantities when required, preferably in amounts of 10 % or less and more preferably in amounts of 3 % or less and most preferred 1 % or less, based on the sum of all residues R selected from the group consisting of hydrogen, methoxymethyl, ethoxymethyl, n-propoxymethyl and iso-propoxymethyl; i.e. on m. If the polyether of the present invention comprises higher homologues of methoxymethyl, where R is C4-alkoxymethyl or higher, the same proviso for the presence of hydrogen applies with the same preferred amounts. Various other co-monomers may be also used in small amounts, i.e. preferably less than 3 wt%, more preferably less than 1 wt% and most preferred in less than 0.1 wt% based on the weight average molecular weight of the polymer of formula [I], if necessary. They may for example be used to introduce functional groups or side chains into the polymer. Best suited for the use in pharmaceutical application are however usually polymers of the present invention that comprise only the repeating units of formula (I).

As mentioned before, ethylene oxide repeating units and glycidyl methyl ether repeating units, i.e. wherein R is methoxymethyl, provide polymers with very similar properties. Because of the very similar properties, the ethylene oxide repeating units in the backbone of PEG may be completely substituted by methoxymethyl substituted repeating units, i.e. 100% of the residues R may be selected from methoxymethyl groups, which constitutes the homopolymer of glycidyl methyl ether. This is however not preferred. The properties of the polymers of the present invention are still closer to the properties of PEG, when at least some of the residues R are selected to be hydrogen. Furthermore, immunogenicity of copolymers will be reduced as compared to the homopolymer of glycidyl methyl ether, because they do not only differ in the orientation of side groups, but also side groups themselves. Preferred are therefore polymers for the present invention that are copolymers. The largest differences between the side groups and therefore the lowest immunogenicity is provided by polymers in which some of the repeating units have no side groups, i.e. where R is hydrogen. Most effective are therefore polymers of the present invention wherein 3 to 99% of the residues R are hydrogen and 1 to 97% of the residues R are independently from each other selected from the group consisting of methoxymethyl, ethoxymethyl, n-propoxymethyl and iso-propoxymethyl.

More preferred are 5 to 95% of the residues R hydrogen and 5 to 95% of the residues R are independently from each other selected from the group consisting of methoxymethyl, ethoxymethyl, n-propoxymethyl and iso-propoxymethyl. This will ensure a lower immunogenicity. More preferred 10 to 90% of the residues R are hydrogen and 10 to 90% of the residues R are independently from each other selected from the group consisting of methoxymethyl, ethoxymethyl, n-propoxymethyl and iso-propoxymethyl. Even more preferred are 20 to 80% of the residues R are hydrogen and 20 to 80% of the residues R are independently from each other selected from the group consisting of methoxymethyl, ethoxymethyl, n-propoxymethyl and iso-propoxymethyl. The lowest immunogenicity provide polymers of the present invention, where about 50 % of all ethylene oxide repeating units are substituted by alkyl glycidyl ethers. For this reason polymers are preferred where 30 to 70% of the residues R are hydrogen and 30 to 70% of the residues R are independently from each other selected from the group consisting of methoxymethyl, ethoxymethyl, n-propoxymethyl and iso-propoxymethyl and more preferred 40 to 60%. Especially useful in pharmaceutical applications is a polyether polymer of the present invention, wherein the polyether is a poly(glycidyl methyl ether-co-ethylene oxide) copolymer, i.e. wherein the residue R is selected from the group consisting of hydrogen and methoxymethyl. The same preferred ranges apply to embodiments where R is selected from the group consisting of hydrogen and methoxymethyl, i.e. wherein the polyether is a poly(glycidyl methyl ether-co-ethylene oxide) copolymer.

Especially preferred are polyether polymer of the present invention, wherein 30 to 70% of the residues R are hydrogen and the remaining residues R are methoxymethyl. These copolymers comprising repeating units where R is hydrogen combine most of the aforementioned advantageous properties. They have low immunogenicity, good water solubility, low crystallinity and are otherwise very similar to PEG.

m is preferably in the range of 20 to 900 and more preferably in the range of 30 to 800. Most preferably m is in the range of 30 to 700. These ranges correspond to the polymer weights of PEG that are most suited for medical applications and that are used in existing medical application for PEG. Furthermore, the polymers of the present invention have preferably a molecular weight $M_n$ as determined by MALDI-TOF in the range of 500 to 50.000 g/mol, more preferably in the range of 1.000 to 50.000 g/mol and most preferably in the range of 1.000 to 30.000 g/mol. These ranges apply to all embodiments of the present invention.

Copolymers of the present invention may be random copolymers. Such copolymers provide the lowest immunogenicity, as they do not provide a blueprint for the immune system for antibodies. They are intrinsically resistant to an immune response and are therefore preferred embodiments of the present invention. These polymers may be prepared by a process of the present invention as discussed below.

In one embodiment of the present invention the polymers of the present invention may be block copolymers or may have a block like structure or a tapered or gradient structure. The methods to prepare such polymers are known to the expert in the art. In such embodiments it is preferred that no more than 5% of the macromolecules of the polymers comprise blocks with more than 15 ethylene oxide repeating units, more preferably that no more than 5% of the macromolecules of the polymers comprise blocks with more than 8 ethylene oxide repeating units. This reduces immunogenicity.

As described above, similarity to PEG is an important feature of the polymers of the present invention. This is especially true for the end groups of the polymer. Processing of PEG for the use in the pharmaceutical field, involves regularly the modification of the end groups. For this purpose, any end group known to be used for PEG can also be used for the present polymers. This includes not only the use of all known end groups for PEG, but also their modification including the kind of modification and the process of modification. If necessary, in order to allow the present polymers to function in this regard as similar or in fact identical to PEG, the present polymers may be provided with ethylene oxide repeating units on either end or both ends of the polymer, as depicted in Formula [Ia] to [Ic]. These are preferred embodiments of the present invention.

$$-(CH_2CH_2\text{-}O\text{-})_a\text{-}(CH_2CHR\text{-}O\text{-})_m\text{-} \quad [Ia]$$

$$-(CH_2CHR\text{-}O\text{-})_m\text{-}(CH_2CH_2\text{-}O\text{-})_a\text{-} \quad [Ib]$$

$$-(CH_2CH_2\text{-}O\text{-})_a\text{-}(CH_2CHR\text{-}O\text{-})_m\text{-}(CH_2CH_2\text{-}O\text{-})_a\text{-} \quad [Ic]$$

In these formulae the partial formulae $-(CH_2CHR\text{-}O\text{-})_m-$ depicts a polymer of the present invention as described herein and a may be any number, but is preferable a number in the range of 1 to 10 and more preferably in the range of 1 to 3. Polymers according to formulae [Ia] to [Ic] allow the modification of the termini of the present polymers exactly as PEG is modified in known processes and especially in present industrial processes. This allows for a smooth transition of established processes from PEG to the polymers of the present invention. Such can be prepared without much additional effort, as will be discussed below.

A further embodiment of the present invention, are polyether polymers represented by any of the following formula [II] and [IIa] to [IIc]:

$$X\text{-}(CH_2CHR\text{-}O\text{-})_m\text{-}CH_2CHR\text{-}Y \quad [II]$$

$$X\text{-}(CH_2CH_2\text{-}O\text{-})_a\text{-}(CH_2CHR\text{-}O\text{-})_m\text{-}CH_2CHR\text{-}Y \quad [IIa]$$

$$X\text{-}(CH_2CHR\text{-}O\text{-})_m\text{-}(CH_2CH_2\text{-}O\text{-})_a\text{-}CH_2CH_2\text{-}Y \quad [IIb]$$

$$X\text{-}(CH_2CH_2\text{-}O\text{-})_a\text{-}(CH_2CHR\text{-}O\text{-})_m\text{-}(CH_2CH_2\text{-}O\text{-})_a\text{-}CH_2CH_2\text{-}Y \quad [IIc]$$

wherein m is 19 to 999 and R is defined as in formula [I] and a is as defined in any of formulae [Ia] to [Ic]. X is the end group derived from the initiator of the reaction and the $\alpha$-end group (alpha end group) and Y is the $\omega$-end group (omega-end group).

X or Y or both may be chosen from any end group known for use in PEG. The same end groups may also be provided for the polymers of formulae [Ia] to [Ic].

X and Y may independently of each other or both comprise one or more functional group selected from the group consisting of acetal (dialkoxy), aldehyde (formyl), amide (carboxamido), azide, carbonate (alkoxycarbonyl)oxy), carboxyl (carboxy), carboxylic anhydride, ester (alkoxycarbonyl), ether, halo, haloformyl (carbonohaloridoyl), hemiacetal (alkoxyol), hemiketal (alkoxyol), hydroxy, imide (imido), imine (imino), ketal (dialkoxy), ketone (oyl), orthoester (trialkoxy), primary, secondary, tertiary amino group, primary, secondary and tertiary alkoxy group, sulfhydryl (sulfanyl, H-S-), thioether and combinations thereof. In a preferred embodiment X or Y may be selected from the group consisting of alkyl, hydrogen, hydroxy, alkoxy, sulfanyl, phthalimide, amide, amine and combinations thereof. X or Y or both may be a primary alkoxy group selected of the formulae $R\text{-}CH_n\text{-}O\text{-}$, wherein R is linear, branched or cyclic alkyl or phenyl and n equals 1 to 20. Preferred are X or Y or both selected from the group consisting of methoxy, ethoxy, propoxy, butoxy, pentoxy, hexoxy, heptoxy, octoxy, nonoxy, decanoxy, 3-ethyl-butoxy, 2,3-dialkoxypropoxy and a combination thereof. X or Y or both may also be the alkoxy residue of an alcohol of the following formula:

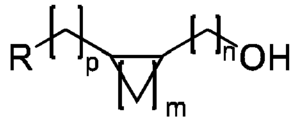

n = 1-20
m = 1-20
p = 0-20

,

wherein R is hydrogen or linear, branched or cyclic alkyl or phenyl. X or Y or both may be the alkoxy residue of a secondary alcohol selected from any of the formulae

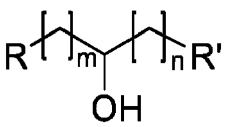

n = 1-20
m = 1-20

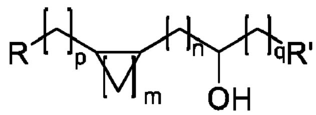

n = 1-20
m = 1-20
p = 0-20
q = 1-20

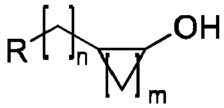

n = 0-20
m = 1-20

,

wherein R and R' are independently from each other hydrogen or linear, branched or cyclic alkyl or phenyl. Among them a secondary alkoxy group is preferred that is selected from the group consisting of 2-propoxy, 2-butoxy, 2-pentoxy, 3-pentoxy, 2-hexoxy, 3-hexoxy, 2-heptoxy, 3-heptoxy, 4-heptoxy and cyclohexyloxy.

X or Y or both may also be selected from primary alkenyloxy groups, which may be linear, branched or cyclic. X or Y or both may also be the alkoxy residue of an alcohol of the following formula:

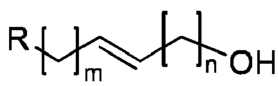

n = 1-20
m = 0-20

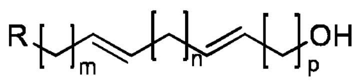

n = 0-20
m = 0-20
p = 1-20

,

wherein R is hydrogen or linear, branched or cyclic alkyl or phenyl. Among them a primary alkenyloxy group is preferred that is selected from the group consisting of 2-propenoxy (allyloxy), 3-butenoxy, 2-butenoxy, 3-methyl-2-butenoxy, 4-pentenoxy, 3-pentenoxy, 2-pentenoxy and 3,5-hexadienoxy.

X or Y or both may further be selected from secondary alkenyloxy groups, which may be branched or cyclic. X or Y or both may be the alkoxy residue of any of the following formulae:

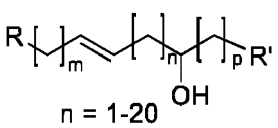

n = 1-20
m = 0-20
p = 1-20

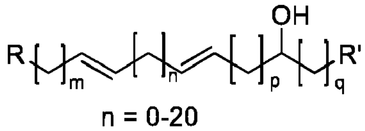

n = 0-20
m = 0-20
p = 1-20
q =1-20

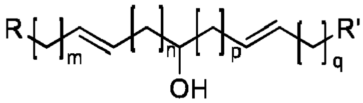

n = 1-20
m = 0-20
p = 1-20
q = 0-20

,

wherein R and R' are independently from each other hydrogen or linear, branched or cyclic alkyl or phenyl. The secondary alkenyloxy group may be of any of the formulae 4-penten-2-oxy, 3-penten-2-oxy, 5-hexen-2-oxy, 4-hexen-2-oxy, 3-hexen-2-oxy, 4,6-heptadien-2-oxy, 1,4-pentadien-3-oxy.

X or Y or both may further be selected from aryloxy and heteroaryloxy groups. Benzyloxy groups are especially preferred. X or Y or both may be the aryloxy or benzyloxy residue of an alcohol of any of the following formulae:

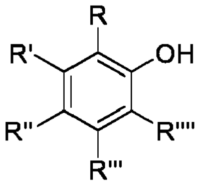
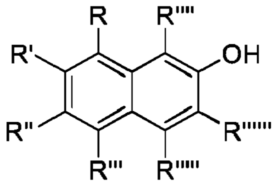
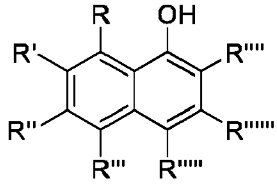
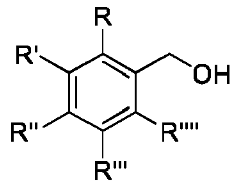

wherein R, R", R"', R"" R"'" and R""" are independently from each other hydrogen or linear, branched or cyclic alkyl or phenyl. X or Y or both are preferably selected from the group consisting of benzyloxy, phenyloxy and naphthyloxy.

X or Y or both may be multifunctional alkoxy groups, i.e. may have multiple ether bonds to the $\alpha$- or $\omega$-position of a plurality of macromolecules of the polymers of the present invention according to formulae [II] or [IIa] to [IIc]. Any suitable polyol may be selected therefore. X or Y or both may especially be selected from the group consisting of polyethers of carbohydrates, especially of Ribose, polyethers of 1,1,1-trimethylolpropane, polyethers of glycerol, polyethers of saccharides or hydrogenated saccharides.

Further, Y may be selected from the group consisting of acrylamide, acrylates, aldehyde, alkyne, amine, aminooxy, azide, benzotriazole carbonate, carboxylic acid, chloroformate, cyanuric chloride, dithioester, epoxide, fluorescein, hydrazide, imidazoyl formate, iminoester, isocyanate, maleimide, mesylate, methacrylate, NHS ester, nitrobenzoate, nitrophenylcarbonate, succinimidyl active ester, succinimidyl carbonate, N-succinimidyl carbonate, succinimidyl succinate, thiocarbonate, thiol, thiol carboxylic acid, tosylate, triflate, vinyl sulfone and xanthate.

It is especially preferred that X is selected from the group consisting of hydrogen, alkyl, hydroxy, sulfanyl, C1-C10-alkoxy, C1-C10-thioalkoxy, amino, amino-Cl-ClO-alkoxy, dibenzylamino-Cl-ClO-alkoxy, amide, N-heterocyclic carbenes and N-heterocyclic olefins. It is also especially preferred that Y is selected from the group consisting of hydrogen, hydroxy, alkoxy, $-CH_2-C(=O)-R$ wherein R is as defined above, -CHO, alkylcarbonyloxy, alkyoxycarbonyloxy, amine, alkylamine, carboxamido, azide, halogen, sulfanyl, thioalkoxy and sulfonate. In addition, for any alkoxy rest defined herein for the residue X the corresponding thioalkoxy residue may be used, i.e. where a sulfur atom is present instead of the oxygen atom.

Most preferred are polymers of the present invention, where X and/or Y are selected from the group consisting of low molecular weight drugs, nanocarriers, liposomal structures, peptides, polypeptides, glycoproteins, polynucleotide, polysaccharides, lipid structures, liposomes, surfaces and interfaces, which are bonded directly by a covalent bond or by a spacer to the polymer. Nanocarriers usable in the present invention are for example selected from the group consisting of carbon nanotubes (CNT), dendrimers, gold nanorods, lipid nanoparticels, liposomes, micelles, nanocrystals, niosomes, polymeric nanoparticles (PNP), solid lipid nanoparticles (SLNs) and virus-based nanoparticles (VNP).

The polymers of the present invention have dispersities of 1.15 or less. They are suitable for applications where low dispersities are required, as for example in pharmaceutical, medical and biological applications. Dispersity of polymers in pharmaceutical, medical and biological applications is of great importance, as macromolecules of different molecular weight may behave differently in biological systems. They may for example differ in solubility in water, membrane permeability, crystallinity and renal clearance. Since a uniform behavior of all molecules is desired in medical applications, a low dispersity is often required. Most preferred are therefore polymers of the present invention that have a dispersity of 1.10 or less. This applies to all embodiments of the present invention. A dispersity of 1.10 or less is required for most medical and pharmaceutical applications. Most preferred are polymers of the present invention which have a dispersity that is lower than 1.08.

Preferred embodiments of the present invention are polyether polymers represented by the following formula [II]:

$$X\text{-}(CH_2CHR\text{-}O\text{-})_mCH_2CHR\text{-}Y \qquad [II]$$

wherein residues R are independently from each other selected from the group consisting of hydrogen, methoxymethyl, ethoxymethyl, n-propoxymethyl and iso-propoxymethyl; 1 to 100% of the residues R are methoxymethyl; up to 50% of the residues R may be selected from the group consisting of ethoxymethyl, n-propoxymethyl and iso-propoxymethyl; with the proviso that at least one residue R is hydrogen, if at least one residue R is selected from the group consisting of ethoxymethyl, n-propoxymethyl and iso-propoxymethyl; X is selected from the group consisting of hydrogen, alkyl, hydroxy, sulfanyl, C1-C10-alkoxy, C1-C10-thioalkoxy, amino, amino-Cl-ClO-alkoxy, dibenzylamino-Cl-ClO-alkoxy, amide, N-heterocyclic carbenes and N-heterocyclic olefins; Y is selected from the group consisting of hydrogen, hydroxy, alkoxy, $-CH_2-C(=O)-R$ wherein R is as defined above, -CHO, alkylcarbonyloxy, alkyoxycarbonyloxy, amine, alkylamine, carboxamido, azide, halogen, sulfanyl, thioalkoxy, N-succinimidyl carbonate and sulfonate; X and/or Y may also be selected from the group consisting of low molecular weight drugs, nanocarriers, liposomal structures, peptides, polypeptides, glycoproteins, polynucleotide, polysaccharides, lipid structures, liposomes, surfaces and interfaces, which are bonded directly by a covalent bond or by a spacer to the polymer, m is in the range of from 19 to 999, characterized in that the dispersity is 1.15 or less. Especially preferred is N-succinimidyl carbonate.

An especially preferred embodiment of the present invention is a polyether polymer of the present invention, wherein X is alkoxy and Y is hydroxy. Further preferably X is a C1-C10-alkoxy group and Y is hydroxy and even more preferably X is a C1-C3 linear alkoxy group and Y is hydroxy. These embodiments are analogous to commercially available PEG derivates. Most preferred X is methoxy and Y is OH, which is analogous to mPEG.

As discussed in regard to the dispersity, a unified behavior of all molecules in drugs and excipients used in pharmaceutical and medical applications is preferred. The end-group fidelity of the polymers of the present invention may be determined by MALDI TOF or by a combination of MALDI TOF with $^1$H NMR by known methods. The polymers of the present invention have preferably an end-group fidelity of at least 95 % in regard to the group X and more preferably of at least 98 %. In a further embodiment the polymers of the present invention have preferably an end-group fidelity of at least 95 % in regard to the group Y and more preferably of at least 98 %. Even more preferred are polyether polymers of the present invention, wherein the end-group fidelity of the polymer in regard to group X and group Y is at least 95% and most preferably of at least 98 %. Especially preferred are polyether polymers of the present invention, wherein the end-group fidelity of the polymer in regard to group X and group Y is at least 95% and the dispersity is 1.10 or less and most preferred wherein the end-group fidelity of the polymer in regard to group X and group Y is at least 98% and the dispersity is 1.10 or less.

As aforementioned, the polymers of the present invention have similar properties as PEG, especially in biological systems. The present polymers may therefore substitute PEG in most of his applications. The polymers of the present invention are especially designed for use in the pharmaceutical and medical field. They may also be used with the same benefits in veterinary medicine and in other biological applications. In these fields the polymers of the present invention can fulfill the same functions as PEG, but have reduced immunogenicity and antigenicity. Because of their irregular structure, they do not elicit a strong immune response. The inventive polymers show especially a low response to anti-PEG antibodies, as evidenced by ELISA tests. Conjugates used for masking drugs and other pharmaceutically active compounds usually comprise PEG in addition to a physiologically active compound or an adjuvant. The polymers of the present invention may be used instead of PEG in any such application. At the same time the immune response elicited by the present polymers will be weak or non-existent thereby resolving one of the main problems in the use of PEG.

A further embodiment of the present invention is therefore a conjugate comprising a polyether polymer of any of the preceding claims and a substrate. The conjugates of the present invention may in the following also be named polyether-conjugated substrates or polymer-conjugated substrates. In the conjugates of the present invention a polymer of the present invention is bonded to a substrate directly or indirectly by a covalent bond. The polymer of the present invention may be bonded to the substrate by a spacer, which constitutes an indirect bond. The substrate may be selected from the group consisting of a pharmaceutically active compound, an adjuvant, a surface and interfaces. The pharmaceutically active compound is preferably selected from the group consisting of low molecular weight drugs, peptides, polypeptides, proteins, glycoproteins, polynucleotides and polysaccharides. The adjuvant is preferably a vesicle or carrier, which are preferably used to carry a pharmaceutically active compound. The adjuvant is preferably selected from the group consisting of nanocarriers, liposomal structures, lipid structures and liposomes. Nanocarriers usable in the present invention are for example selected from the group consisting of carbon nanotubes (CNT), dendrimers, gold nanorods, lipid nanoparticels, liposomes, micelles, nanocrystals, niosomes, polymeric nanoparticles (PNP), solid lipid nanoparticles (SLNs) and virus-based nanoparticles (VNP). In a further preferred embodiment the substrate is selected from the group consisting of low molecular weight drugs, nanocarriers, liposomal structures, peptides, polypeptides, proteins, glycoproteins, polynucleotides, polysaccharides, lipid structures, liposomes, surfaces and interfaces. Also in these embodiments the substrate may be bonded directly by a covalent bond or by a spacer to the polymer. Especially preferred are conjugates of the present invention with bovine serum albumin.

The polymer of the present invention may be bonded to the substrate directly by a covalent bond or by a spacer or any kind. Preferably a hydroxy-residue at the $\omega$-terminal of the polymer of the present invention is used to bond a substrate or a spacer to the polymer. This is most economical, as the polymers of the present invention where X is an inert group and Y is OH can be most easily be prepared (see Examples). The conjugation of a substrate to the OH group at the $\omega$-terminal end of PEG is well established. The same processes may be used for the polymers of the present invention. The substrate may however also be bonded to the $\alpha$-terminal of the polymer. The polymers of the present invention can also be bonded to more than one substrate. This can be done by use of the $\alpha$-terminal and the $\omega$-terminal of the polymer and by using functional end groups of the polymer or spacer that can bond to a plurality of substrates. This may also be achieved by use of co-monomers with functional groups to which a substrate may be bonded. The bond between the polymers of the present invention and the substrate may be prepared by any means currently used for bonding PEG to substrates. It is also possible to bond a plurality of macromolecules of the polymers of the present invention to one substrate. This is especially useful for vesicle of all kinds including liposomes.

Typical examples for PEG conjugates are the lipid nanoparticles that are essential for vaccines against Covid-19 of the firms BioNTech SE and Pfizer Inc. and Moderna. It comprises a PEG conjugate of lipids, and the lipid nanoparticles (LNPs) are used to encapsulate and protect mRNA, which is the pharmaceutically active substance. The PEG in these conjugates may be substituted by the polymers of the present invention, thereby retaining the effects of the PEG polymer in the conjugate and at the same time reducing the immune response to the conjugate. A further vaccine of this kind is the Covid-19 vaccine of Curevac. Further drugs that comprise PEG conjugates wherein the PEG could be substituted by the present polymers are listed in the following.

Table 1

| Trade names | Chemical Structure | Indication |
|---|---|---|
| Jivi® | 60k-PEG recombinant Factor VII antihemophilic factor | Hemophilia A |
| Palynziq® | 2K-PEG-rhu-Phenylalanine ammonia-lyase, Peqvaliase-pqpz | Phenyl-ketonuria |
| Adynovate® | 20K-PEG-Factor VIII Antihemophilic Factor VIII | Hemophilia A |
| Revolixys® kit | 40K-PEG-RNA aptamer + reverse agent, Factor-IXa blocker, Pegnivacogin /Anivamersen | Anti-coagulation |
| Onicyde® | 2K-PEG-Liposomal irinotecan hydrochloride trihydrate | Metastatic pancreatic cancer |
| Plegridy® | 20k PEG-Interferon β-1a | Relapsing from multiple sclerosis |
| Movantic® | <1k PEG-Naloxegol | Opioid-induced constipation |
| Omontys® | 40K-PEG-Erythropoietin-mimetic peptide, Peginesatide | Anemia associated with chronic kidney disease |
| Sylatron® | 12K-PEG-Interferon α-2b | Melanoma |
| Krystexxa® | 10k-PEG-Uricase, Peqloticase | Gout |
| Cimzia® | 40k-PEG-Certolizumab | Rheumatoid arthritis, Crohn's disease, Axial spondyloarthritis and psoriatic arthritis |
| Mircera® | 30K-PEG- erythropoietin (epoetin) β | Anemia associated with chronic kidney disease |
| Macugen® | 40K-PEG-anti-VEGF aptamer, Pegaptanib | Age-related macular degeneration |
| Somavert® | 5K-PEG-rhuGH (human growth hormone), Pegvisomant | Acromegaly |
| Neulasta® | 20K-PEG-Granulocyte colony stimulating factor, Pegfilgrastim | Neutropenia |
| Pegasys® | 40K-PEG-interferon α-2 | Hepatitis C und B |
| PegIntron® | 12K-PEG-interferon α-2b | Hepatitis C und B |
| Doxil®/Caelyx® | 2K-PEG-Liposomal doxorubicin HCl | Cancer |
| Oncaspar® | 5K-PEGylated L-asparaginase, Pegaspargase | Acute lymphoblastic leukemia |
| Adagen® | 5K-PEG-adenosine deaminase (bovine), Pegademase | Severe combined immunodeficiency disease (SCID) |

(continued)

| Trade names | Chemical Structure | Indication |
|---|---|---|
| Irinotecan® | PEGylated liposomal irinotecan hydrochloride trihydrate | Metastatic pancreatic cancer |
| Lonquez® | 20k PEG-rhG-CSF | Neutropenia |
| Jintrolong®[1] | Branched 40k PEG hGH | hGH deficiency |
| Neulapeg® | 20k-PEG rhG-CSF mutein | Neutropenia |
| Rebinyn®/ Refixia® | Branched 40k PEG Recombinant factor VIII | Hemophilia B |
| Asparlas® | 5k-PEG L-Asparaqinase | Acture lymphoblastic leukemia |
| Revcovi® | 5k-PEG Recombinant B-domain truncated factor VIII | Hemophilia A |
| Besremi®[3] | Branched 40k PEG Proline-interferon α-2b | Polycythemia vera |
| PEGPH20®[3] | 30k-PEG recombinant human hyaluronidase | Pancreatic cancer |
| Pegargiminase | 20k PEG Arginine deiminase Interleukin-2 | Hepatocellular carcinoma |
| NKTR-214 | Branched PEG-20k Fmoc-succinimidyl carbonate | Melanoma, rencal cell carcinoma |
| PRX-201[3] | Bifunctional PEG2k α-Galactosidase A | Fabry disease |
| Peginterferon lambda-la | 20k PEG recombinant interferon lambda-la | Hepatitis C and B[2], Hepatitis D[3] |
| Abicipar pegol | 20k PEG anti-VEGF DARPin | Neovascular age related macular degeneration |
| TransCon GH®[3] | Four-arm-40PEG spacer hGH | Growth hormone deficiency |
| AM0010[2] | 30k PEG-Interleukin-10 | Metastatic pancreatic cancer |
| Rolontis®[3] | Bifunctional PEG rhG-CSF | Neutropenia |
| Efpeglenatide[3] | Bifunctional PEG Exendin-4 (glucagon-like peptide-1 receptor agonist) | Type-2 diabetes mellitus |
| Pegbelfermin[2] | 30k PEG fibroblast growth factor-21 mutein | Fibrosis, liver disorders, nonalcoholic steatohepatitis, type 2 diabetes mellitus |
| Imrestor® (veterinary use) | 20k PEG recombinant bovine G-CSF mutein | Reducing risk of clinical mastitis |
| Pegfilgrastim biosimilars: Fulphila (Mylan®) Lapelga/Pelgraz (Apobiologix®), Udenyca (Coherus Biosciences®), Pelmeg (Mundipharma®), Ziextenzo (Sandoz@) | 20K-PEG-Granulocyte colony stimulating factor, Pegfilgrastim, rhG-CSF | Neutropenia |

(continued)

| Trade names | Chemical Structure | Indication |
|---|---|---|
| Esperoct® | Branched-PEG40k-SA-CMP, enzymatic glycopegylation at O-glycan on Ser-750 | Hemophilia A |
| [1] only in China<br>[2] Phase II<br>[3] Phase III | | |

Process for the preparation of a polyether polymer of the present invention, by anionic ring-opening copolymerization (AROP) comprising the steps of:

- Providing an anion $An^-$,
- Adding at least one monomer of the formula 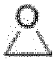 -R, wherein R is as defined as described for any of formulae [I] or [II],
- Allowing the polymerization to proceed at a temperature in the range of -10 to 90 °C

wherein the monomer comprises less than 1 wt% of epichlorohydrin.

In the process of the present invention the anion $An^-$ must be an anion suitable for anionic ring-opening copolymerization (AROP). The expert in the art can easily choose among the known anions for this purpose. The $An^-$ anion may be selected from the groups consisting of alkyl anion, hydride, $OH^-$, alkoxy, $SH^-$, thioalkoxy, amine anion, amide anion, imide anion and combinations thereof. Alkyl anions and hydride anions may be provided in the form of metal alkyl or metal hydride compounds. The alkoxy anion and the thioalkoxy anion may be the corresponding anion with the same structure as any of the alkoxy groups and aryloxy groups and aralkoxy groups defined herein for the residue X. Preferably however the alkoxy anion and the thioalkoxy anion are not tertiary alkoxy anions. The imide anion is preferably a phthalimide anion. Polymers of the Formulae [Ia], [Ic], [IIa] and [IIc] may be prepared by first adding the corresponding amount of ethylene oxide to the anion $An^-$ and allowing the polymerization to proceed at a temperature in the range of -10 to 90 °C until all of the ethylene oxide is consumed. Subsequently the steps of adding least one monomer of the formula 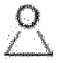 -R, and allowing the polymerization to proceed at a temperature in the range of -10 to 90 °C are performed. In this manner the repeating units adjacent to the $\alpha$-terminal of the polymers of the present invention are provided with exactly the same structure as PEG. Polymers of the Formulae [Ia], [Ic], [IIa] and [IIc] may also be prepared by use of anions of the formulae $X\text{-}(CH_2CH_2\text{-}O\text{-})_a^-$, wherein a is as defined for any of the Formulae [Ia], [Ic], [IIa] and [IIc] and X is any residue as defined herein for X. This is the preferred method. Especially preferred is the use of such anions of the formulae $X\text{-}(CH_2CH_2\text{-}O\text{-})_a^-$, in which X is alkyl, alkoxy, dialkylamin anion or $(RCO)_2N^-$. Even more preferred is the use of anions selected from the group consisting of $MeOCH_2CH_2O^-$, $MeO(CH_2CH_2O)_2^-$, $BenzylOCH_2CH_2O^-$, $BzO(CH_2CH_2O)_2^-$, $(Bz)_2N\text{-}CH_2CH_2O^-$, $(Bz)_2N\text{-}(CH_2CH_2O)_2^-$, phthalimide-$CH_2CH_2O^-$, phthalimide-$(CH_2CH_2O)_2^-$, wherein Me is methyl and Bz is benzyl. Most preferred are $MeO(CH_2CH_2O)_2^-$, $BzOCH_2CH_2O^-$ and $(Bz)_2N\text{-}CH_2CH_2O^-$.

Further, in a final step of the preparation, addition of a small amount of pure EO is possible to ensure primary hydroxyl end groups for the $\omega$-terminal of the polymer that can undergo all established coupling strategies to therapeutic entities. This leads to polymers of the present invention with Formulae [Ib], [Ic], [IIb] and [IIc].

The counter ion to the $An^-$ anion is preferably selected from the group consisting of $Na^+$, $K^+$ and $Cs^+$. The anion $An^-$ may be provided in an inert solvent. The solvent is preferably a non-protic solvent and most preferably dimethyl sulfoxide (DMSO). Also preferably the reaction is performed in the same solvent.

Anionic ring-opening polymerizations with alkyl glycidyl ethers are more prone to undergo chain transfer reactions as follows:

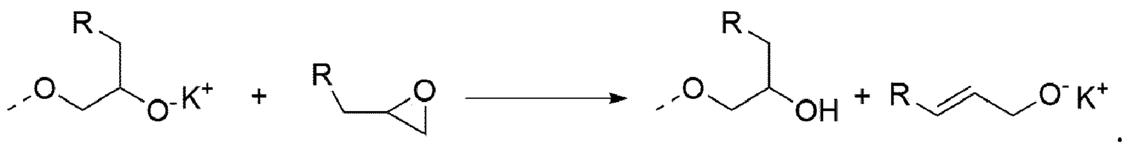

These side reactions increase with the temperature. Therefore, high molecular weight polymers can only be obtained, if the reaction temperature is not above 90°C. The chain transfer reaction results in a broad distribution of molecular weight and therefore in a high dispersity. Therefore, lower polymerization temperatures also provide polymers of the present invention with lower dispersity. On the other hand, alkyl glycidyl ethers are as reactive in the anionic ring-opening polymerization as ethylene oxide. For this reason the polymerization may be performed at temperatures as low as -10°C. Preferably the polymerization is performed at a temperature in the range of 10 to 70°C and more preferred at a temperature in the range of 10 to 60°C.

A further problem in anionic ring-opening polymerizations is that commercially available alkyl glycidyl ethers are prepared from epichlorohydrin. Part of the epichlorohydrin remains in the alkyl glycidyl ether. Presence of alkyl glycidyl ethers leads to termination reactions as follows:

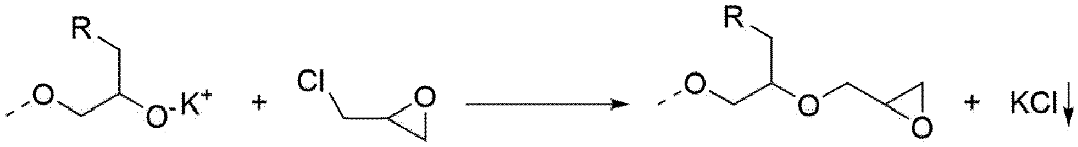

This in turn leads to low molecular weights and high dispersity of the product. The polymers of the present invention are therefore only accessible by reaction temperatures of -10 to 90°C or less and by the use of alkyl glycidyl ethers that comprise less than 1 wt% of epichlorohydrin. Preferably the alkyl glycidyl ethers comprise less than 0.5 wt% of epichlorohydrin and more preferably less than 0.1 wt%. Most preferably the alkyl glycidyl ether are free of epichlorohydrin. Removal of epichlorohydrin from the glycidyl methyl ether to the extent necessary is impossible, because the glycidyl methyl ether and epichlorohydrin have similar boiling points, e.g. epichlorohydrin 117.9°C, glycidyl methyl ether 110°C. Procedures for the preparation of epichlorohydrin free alkyl glycidyl ethers is provided in Example 1 of the present invention.

Furthermore, high temperatures also lead to side reactions of the starting anion $An^-$ or the group X of the living polymer. Limitation of the temperature in the aforementioned range therefore also improves the end-group fidelity at the $\alpha$-terminal of the polymers of the present invention and allows for end-group fidelities of 95% or more of the X residue. Other known methods for the polymerization of the alkyl glycidyl ethers do not provide the low dispersity required for the present polymers. They also do not allow for the same high end-group fidelity.

In order to provide random copolymers or the present invention, it is necessary to add at least two different kinds of monomers to the anion. The reactivity ratio for both alkyl glycidyl ether employed and ethylene oxide is in the range of 0.7 to 1.3. For glycidyl methyl ether and ethylene oxide the reactivity ratio is in the range of 0.98 to 1.02. This results in almost ideally random copolymers. Other forms of copolymers i.e. with a block structure, block like structure or a tapered or gradient structure can be prepared according to known methods. Block copolymers can easily be prepared by subsequent additions of different monomers to the living polymer. Therefore, a process of the present invention is preferred, wherein the step of adding at least one monomer and the step of allowing the polymerization to proceed are repeated at least one time, whereby at least one different monomer is used than the first time.

A further embodiment of the present invention is the use of the polyether polymers of the present invention for the preparation of a conjugate of the polymers with a substrate. The substrate is as defined herein for the conjugates of the present invention. The substrate is preferably a bioactive compound. There is a well-developed chemistry for the preparation of conjugates of PEG, which is known to the expert in the art. The same chemistry may be used for the preparation of a conjugate of the present invention. Preferred is a use of the present invention, for the preparation of conjugated lipids for use in vaccines, in particular based on lipid nanoparticles. These nanoparticles are preferably nanoparticles, as used against COVID-19. Preferred are conjugates of polymers of the present invention with bovine serum albumin (BSA). Especially preferred are conjugates with polymers of the present invention where the y group of the polymer is a N-succinimidyl carbonate group. Even more preferred are conjugates of the present polymer where bovine serum albumin in conjugates by means of such a group Y to a polymer of the present invention. Conjugation of $\alpha$-BzO-ω-N-succinimidyl carbonate-P(EG-co-GME) to bovine serum albumin if most preferred.

An additional embodiment for the present invention concerns the preparation of conjugates of the present invention. As discussed above, the conjugates may be prepared by any known process for the preparation of conjugates of PEG. The present invention provides additionally a process, where firstly the process for the preparation of the polymers of the present invention is performed and then a substrate is added to the living polymer, wherein the substrate has a functional group that reacts with the living polymer to form a conjugate.

Brief description of the figures:

Figure 1: Figure 1 shows the results of ELISA test of three of the polymers of the present invention and mPEG.

Figures 2 to 8: These figures show the SEC traces of polymers of the present invention and Figure 8 shows in addition the SEC trace of commercially available mPEG.

Figure 9: Figure 9 shows the $^1$H NMR spectrum of $\alpha$-BzO-P(EG0.51-co-GME0.49).

Figure 10: Figure 10 shows the MALDI-TOF spectrum of $\alpha$-BzO-P(EG0.51-co-GME0.49).

Figure 11: Figure 11 shows cloud point of $\alpha$-BzO-P(EG0.51-co-GME0.49).

Figure 12: Figure 12 shows the MALDI-TOF spectrum of $\alpha$-DBzN-EtO-PGME, i.e. a homopolymer of the present invention.

Figure 13: Figure 13 shows the MALDI-TOF spectrum of $\alpha$-MeO-P(PEG$_{0.88}$-*b*-PGME$_{0.22}$), i.e. a block copolymer of the present invention.

Figure 14: Figure 14 shows the MALDI-TOF spectrum of $\alpha$-BzO-$\omega$-Ts-P(EG$_{0.93}$-*co*-GME$_{0.07}$), i.e. a copolymer of the present invention that has been further functionalized at the $\omega$-terminal ending.

Figure 15: Figure 15 shows the decreasing $^1$H NMR signals of monomers during the preparation of a polymer of the present invention.

Figure 16: Figure 16 shows the result of a study of the relative reactivity of ethylene oxide and glycidyl methyl ether in the anionic ring opening polymerization.

Examples:

Reagents:

Chemicals were purchased from TCI, Acros Organics, Roth, Sigma Aldrich and Honeywell, unless otherwise noted. Ethylene oxide was obtained from Air Liquide. Deuterated solvents were purchased from Deutero GmbH. THF was flashed over basic aluminum oxide before usage. Glycidyl methyl ether was dried over $CaH_2$ and cryo-transferred before the polymerizations.

Measurements:

$^1$H and $^{13}$C NMR spectra were recorded on a Bruker Avance III HD 300 spectrometer with 300 MHz and referenced internally to residual proton signals of the deuterated solvent.

Size-exclusion chromatography measurements were performed with dimethylformamide (DMF with 1 g/L LiBr) as the mobile phase (flow rate 1 mL/min) on 2-hydroxyethylmethacrylat (HEMA) 300/100/40 columns at 50 °C. Polymer concentrations were 1 mg/mL. Calibration was carried out using poly(ethylene glycol) standards (from Polymer Standard Service, Mainz, Germany).

Differential scanning calorimetry (DSC) measurements were carried out in the temperature range of -100 to 100 °C with a heating rate of 10 K/min at a PerkinElmer DSC 8500. The thermal history of the samples was excluded via two cooling and two heating cycles. For each sample, the glass transition and the melting temperatures were obtained from the second heating curve.

MALDI-ToF MS measurements were carried out at a Bruker autoflex maX MALDI-TOF/TOF. The potassium salt of trifluoroacetic acid and trans-2-[3-(4-tert-Butylphenyl)-2-methyl-2-propenylidene]malononitrile (DCTB) were utilized as ionization salt and matrix, respectively.

Example 1: Synthesis of Glycidyl methyl ether (GME)

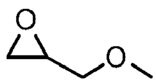

a) Allyl methyl ether (10.0 g, 139 mmol) was dissolved in 274 mL dichloromethane (DCM) and m-chloroperoxybenzoic acid (m-CPBA, 70%, 37.6 g, 153 mmol (based on m-CPBA)) was added to the solution. After stirring overnight, the solution was filtrated and slowly concentrated under reduced pressure. Crude glycidyl methyl ether was separated from solid impurities via cryo-transfer. Slow evaporation of residual dichloromethane in vacuo gave pure glycidyl methyl ether as a colorless liquid; Yield 41%.

b) 1-Chloro-3-methoxy-propan-2-ol (3.00 g, 2.56 mL, 24.1 mmol) and anhydrous sodium sulfate (1.02 g, 7.23 mmol) were added to a flask equipped with a magnetic stirrer and cooled with a water bath. Finely grounded sodium

hydroxide (1.25 g, 31.3 mmol) was added under stirring. After complete reaction (TLC control) the crude product was cryo-transferred in vacuo from the reaction flask and dried over CaH2 under cooling. After an additional cryo-transfer, GME was obtained as a colorless liquid with a yield of 93 %.

Example 2: General procedure for the preparation of random copolymers Synthesis of α-BzO-P(EG-co-GME)

$$Ph\text{-}CH_2\text{-}O\text{-}CH_2\text{-}CH_2\text{-}O\text{-}[CH_2\text{-}CH_2\text{-}O\text{-}]_{98}$$

$$\therefore [CH_2CH(CH_2\text{-}O\text{-}CH_3)\text{-}O\text{-}]_8H$$

Potassium tert-butoxide (13.0 mg, 118 μmol) was dissolved in a tetrahydrofuran (THF) - water mixture and transferred into a reaction flask, equipped with a Teflon stopcock and a septum. 2-Benzyloxy-ethanol (20.0 mg, 18.7 μL, 131 μmol) was dissolved in benzene and added to the reaction flask. After slow evaporation of the solvent, the resulting solid was dried at 60 °C under high vacuum overnight. The residue was dissolved in dimethyl sulfoxide (DMSO, 5 mL) and the solution was cooled to -78 °C. Glycidyl methyl ether (119 mg, 122 μL, 1.35 mmol) was added via syringe and ethylene oxide (537 mg, 553 μL, 12.2 mmol) was cryo-transferred into the reaction flask. The solution was heated to 55 °C and stirred for 24 h under vacuum. Afterwards, the solution was poured into excess chloroform and the organic phase was extracted against water (3 times) and brine, dried over $MgSO_4$ and filtrated. After evaporation of the solvent α-BzO-P(EG0.92-co-GME0.08) was obtained as a colorless solid; yield quantitative.

The data of the polymer produced can be found in Tables 2 to 4 below (see entry b). Further examples have been prepared according to this procedure with varying amounts of the monomers (see Tables 2 to 3, entries a to d, f and g). Further, α-MeO-P(EG-co-GME) and α-DBzN-P(EG-co-GME) were prepared according to the same procedure with diethylene glycol monomethyl ether and N,N-dibenzyl-2-aminoethanol as initiators, respectively, instead of benzyloxy-ethanol (entries h and i of Tables 2 to 4).

Example 3: Synthesis of

$$Ph\text{-}CH_2\text{-}O\text{-}CH_2\text{-}CH_2\text{-}O\text{-}[CH_2\text{-}CH_2\text{-}O\text{-}]_{51} \therefore [CH_2CH(CH_2\text{-}O\text{-}CH_3)\text{-}O\text{-}]_{49}H$$

Cesium hydroxide monohydrate (28.0 mg, 118 μmol) was dissolved in a THF-water mixture and transferred into a reaction flask, equipped with a Teflon stopcock and a septum. 2-Benzyloxy-ethanol (20.0 mg, 18.7 μL, 131 μmol) was dissolved in benzene and added to the reaction flask. After slow evaporation of the solvent, the resulting solid was dried at 60 °C under high vacuum overnight. The residue was dissolved in DMSO (5 mL) and the solution was cooled to -78 °C. GME (367 mg, 374 μL, 4.16 mmol) was added via syringe and EO (183 mg, 189 μL, 4.16 mmol) was cryo-transferred from a graduated ampule into the reaction flask. The solution was stirred for 48 h at room temperature under vacuum. Afterwards, the solution was poured into excess chloroform and the organic phase was extracted against water (3 times) and brine, dried over $MgSO_4$ and filtrated. $P(EG_{0.51}\text{-co-}GME_{0.49})$ was obtained in quantitative yield as a viscous liquid after evaporation of the solvent and drying under high vacuo. The data of the product can be found in Tables 2 and 3, entry e.

Example 4: Synthesis of glycidyl methyl ether homopolymer

$$\alpha\text{-}Bz_2NCH_2CH_2O(CH_2CH(CH_2OCH_3)\text{-}O)_nH$$

Cesium hydroxide monohydrate (25.0 mg, 149 μmol) was dissolved in a THF-water mixture and transferred into a reaction flask, equipped with a Teflon stopcock and a septum. N,N-Dibenzyl-2-aminoethanol (40.0 mg, 166 μmol) was dissolved in benzene and added to the reaction flask. After slow evaporation of the solvent, the resulting solid was dried at 60 °C under high vacuum overnight. The residue was dissolved in DMSO (5 mL) and the solution was cooled to -78 °C. Glycidyl methyl ether (GME, 660 mg, 670 μL, 7.46 mmol) was added via syringe. The solution was stirred for 48 h at room temperature under vacuum. Afterwards, the solution was poured into excess chloroform and the organic phase was extracted against water (3 times) and brine, dried over $MgSO_4$ and filtrated. Poly(glycidyl methyl ether) (PGME) was obtained in quantitative yield as a viscous liquid after evaporation of the solvent and drying under high vacuo. The data of this polymer can be found in Table 2 and 3, entry j.

Example 5: Synthesis of $\alpha$-BzO-P(EG-co-EGE)

$$Ph\text{-}CH_2\text{-}O\text{-}CH_2\text{-}CH_2\text{-}O\text{-}[CH_2\text{-}CH_2\text{-}O\text{-}]_{0.92} \because\ [CH_2CH(CH_2\text{-}O\text{-}CH_2\text{-}CH_3)\text{-}O\text{-}]_{0.08}H$$

Potassium tert-butoxide (13.0 mg, 118 μmol) was dissolved in a THF-water mixture and transferred into a reaction flask, equipped with a Teflon stopcock and a septum. 2-Benzyloxy-ethanol (20.0 mg, 18.7 μL, 131 μmol) was dissolved in benzene and added to the reaction flask. After slow evaporation, the resulting solid was dried at 60 °C under high vacuum overnight. The residue was dissolved in DMSO (5 mL) and the solution was cooled to -78 °C. Ethyl glycidyl ether (EGE, 270 mg, 260 μL, 2.50 mmol) was added via syringe and ethylene oxide (440 mg, 450 μL, 9.99 mmol) was cryo-transferred from a graduated ampule into the reaction flask. The solution was heated to 55 °C and stirred for 24 h under vacuum. Afterwards, the solution was poured into excess chloroform, and the organic phase was extracted against water (3 times) and brine, dried over $MgSO_4$ and filtrated. P(EG0.92-co-EGE0.08) was obtained as a colorless solid after evaporation of the solvent and drying under high vacuo; yield > 96%. Table 2 and 3, entry k shows the data of this product.

Example 6: Synthesis of mPEG-b-PGME

$$Me\text{-}O\text{-}[CH_2\text{-}CH_2\text{-}O\text{-}]_{0.78}\text{-}[CH_2CH(CH_2\text{-}O\text{-}CH_3)\text{-}O\text{-}]_{0.22}H$$

Potassium tert-butoxide (13.0 mg, 118 μmol) was dissolved in a tetrahydrofuran (THF) - water mixture and transferred into a reaction flask, equipped with a Teflon stopcock and a septum. Methoxypoly(ethylene glycol) (mPEG, $M_n$ 2 kg/mol; 250 mg, 125 μmol) was dissolved in benzene and added to the reaction flask. After slow evaporation, the residue was dried at 80 °C under high vacuum overnight. The residue was dissolved in dimethyl sulfoxide (DMSO, 5 mL) and the solution was cooled to -78 °C. Glycidyl methyl ether (253 mg, 258 μL, 2.88 mmol) was cryo-transferred into the reaction flask. The solution was heated to 55 °C and stirred for 24 h under vacuum. Afterwards, the solution was poured into excess chloroform and the organic phase was extracted against water (3 times) and brine, dried over $MgSO_4$ and filtrated. mPEG-b-PGME 4k was obtained as a colorless solid after precipitation in ice-cold diethyl ether; yield quantitative. Table 2 and 3, entry j shows the data for this polymer.

Example 7: Synthesis of $\alpha$-BzO-ω-Ts-P(EG-co-GME)

$$Ph\text{-}CH_2\text{-}O\text{-}CH_2\text{-}CH_2\text{-}O\text{-}[CH_2\text{-}CH_2\text{-}O\text{-}]_{98} \because\ [CH_2CH(CH_2\text{-}O\text{-}CH_3)\text{-}O\text{-}]_8Ts$$

$\alpha$-BzO-P(EG-co-GME) (50.0 mg, 4.6 μmol) from Example 2 was dissolved in dichloromethane (DCM) in a flask equipped with a reflux condenser and the solution was cooled to 0 °C. After addition of triethylamine (2.32 mg, 3.18 μL, 22.9 μmol), tosyl chloride (4.37 mg, 22.9 μmol) was added in portions to the solution. The ice-bath was removed and the solution was refluxed overnight. The reaction mixture was extracted with saturated $NaHCO_3$, water and brine, dried over $MgSO_4$ and filtrated. After precipitation in diethyl ether and drying in vacuo, $\alpha$-BzO-ω-Ts-P(EG-co-GME) was obtained as a white solid; yield quantitative. Tables 2 and 3, entry m shows the data for this polymer.

Example 8: Synthesis of α-BzO-ω-N-succinimidyl carbonate-P(EG-co-GME)

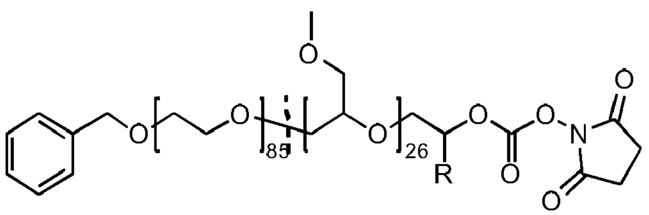

R= H, $CH_2OCH_3$

N,N'-disuccinimidyl carbonate (3.5 mg, 13.8 µmol) is added to a stirred solution of $\alpha$-BzO-P(EG-co-GME) (50.0 mg, 4.6 µmol) from Example 2 in dry $CH_3CN$ (1 ml) at room temperature for 18 h. The reaction mixture was dissolved in dichloromethane extracted with a saturated $NaHCO_3$, water and brine, dried over $MgSO_4$ and filtered. The solvent was removed under reduced pressure and the polymer dried under vacuum. The product was obtained as a white solid; yield quantitative.

Example 9: Conjugation of $\alpha$-BzO-ω-N-succinimidyl carbonate-P(EG-co-GME) to bovine serum albumin (BSA).

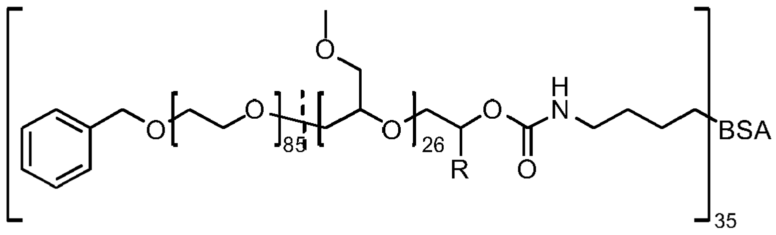

R= H, $CH_2OCH_3$

Bovine serum albumin (5 mg, 0.07 µmol) and α-BzO-ω-N-succinimidyl carbonate-P(EG-co-GME) (16.9 mg, 2.6 µmol) from Example 8 were stirred in phosphate-buffered saline (PBS buffer) for 1 h. The unreacted polymer was removed by dialysis in deionized water with a 25 kDa membrane filter. The conjugate was then dried by lyophilization and obtained in quantitative yield. In the preparation of this conjugate, the outer lysing group (30-35) are targeted for the conjugation.

Example 10: Competitive Enzyme-linked Immunosorbent Assay (ELISA)

The interaction of the copolymer samples entry c, d and e were evaluated by a competitive PEG ELISA kit using a murine monoclonal, horseradish peroxidase conjugated anti PEG antibody (HRP anti-PEG) (Life diagnostics, West Chester, PA, USA). Samples of concentrations ranging from 0 to 4600 µg ml-1 were prepared in dilution buffer. Additionally, mPEG with a molecular weight of 5000 g mol-1 was utilized as internal standard for comparison. 50 µl of each prepared sample was dispensed to a PEG pre-coated 96-well plate and 50 µl of HRP anti-PEG was added to each well. The solutions were incubated for 1h at 25 °C with a micro-plate shaker and then washed six times with 400 µl of wash buffer per each well. After removal of residual droplets, 100 µl of 3,3',5,5'-Tetramethylbiphenyl-4,4'-diamine was added to each well and the solutions were mixed on a micro-plate shaker for 20 min. The reaction was stopped by addition of 100 µl of stop solution and the absorbance at 450 nm was read within 5 minutes.

Analysis of ELISA Data: The determined absorbance values were normalized to visualize the percent of maximal binding. The sample concentrations were transformed to a function of $\log_{10}$. The sigmoidal fits were calculated using the following equation with a representing the upper, b the lower limit, c the inflection point and d the hill slope.

$$y = a + (b-a) / 1 + 10^{(c-x)*d}$$

Example 11: Kinetic of the Synthesis of α-BzO-P(EG-co-GME)

The synthesis of $\alpha$-BzO-P(EG-co-GME) was repeated in fully deuterated DMSO for an online in situ $^1$H NMR kinetic measurement. 95 mg glycidyl methyl ether (95 µl, 1.1 µmol) and 32 mg ethylene oxide (33 µl, 7.1 µmol), i.e. 60% glycidyl methyl ether and 40% ethylene oxide where solved in fully deuterated DMSO. The consumption of ethylene oxide and glycidyl methyl ether over time was measured by $^1$H-NMR spectroscopy. As measure for the ethylene oxide concentration, the signal of the protons of ethylene oxide at about 2.6 ppm was used. For glycidyl methyl ether the signals of the protons at the oxirane ring with absorptions at about 2.75 and 3.1 ppm were used. Figure 15 shows the decrease of the selected $^1$H NMR signals of the two monomers over time. Figure 16 shows plot of monomer consumption Mx,t/Mx,t=0 versus total conversion. As can be seen, ethylene oxide and glycidyl methyl ether are consumed at exactly the same rate. This data shows that the copolymers of the present invention are almost ideal random copolymers.

Discussion of the results

Tables 2, 3 and 4 and Figure 1 to 15 show the properties of the polymers prepared. They are discussed in the

following.

Table 2: Composition of polymers prepared

| *Entry* (Example.) | *Sample* | $DP_{EO}$ NMR | $DP_{GE}$ NMR | $mol\%_{EO}$ NMR | $mol\%_{GME}$ NMR |
|---|---|---|---|---|---|
| a (2) | $\alpha$-BzO-P($EG_{0.96}$-*co*-$GME_{0.04}$) | 108 | 4 | 96 | 4 |
| b (2) | $\alpha$-BzO P($EG_{0.92}$-*co*-$GME_{0.08}$) | 98 | 8 | 92 | 8 |
| c (2) | $\alpha$-BzO P($EG_{0.84}$-*co*-$GME_{0.16}$) | 112 | 21 | 84 | 16 |
| d (2) | $\alpha$-BzO P($EG_{0.76}$-*co*-$GME_{0.24}$) | 85 | 26 | 76 | 24 |
| e (3) | $\alpha$-BzO P($EG_{0.51}$-*co*-$GME_{0.49}$) | 35 | 33 | 51 | 49 |
| f (2) | $\alpha$-BzO P($EG_{0.96}$-*co*-$GME_{0.04}$) | 223 | 10 | 96 | 4 |
| g (2) | $\alpha$-BzO P($EG_{0.93}$-*co*-$GME_{0.07}$) | 175 | 13 | 93 | 7 |
| h (2) | $\alpha$-MeO-P($EG_{0.97}$-*co*-$GME_{0.03}$) | 147 | 5 | 97 | 3 |
| i (2) | $\alpha$-DBzN-P($EG_{0.97}$-*co*-$GME_{0.03}$) | 142 | 5 | 97 | 3 |
| j (4) | $\alpha$-DBzN-EtO-PGME | - | 45 | - | 100 |
| k (5) | $\alpha$-BzO-P($EG_{0.85}$-*co*-$EGE_{0.15}$) | 106 | 18 | 85 | 15 |
| l (6) | $\alpha$-MeO-$PEG_{0.88}$-*b*-$PGME_{0.22}$ | | 50 | 14 | 88 22 |
| m (7) | $\alpha$-BzO-$\omega$-Ts-P($EG_{0.93}$-*co*-$GME_{0.07}$) | | 223 | 10 | 96 4 |

*GE* = Glycidyl ether; *DP* = degree of polymerization

Table 3: Characterization data of polymers prepared

| *Entry*/ (Example) | *Sample* | $M_{n,NMR}$ [kg/mol] | $M_{n,MALDI}$ [kg/mol] | $M_{n,SEC}$ [kg/mol] | $PDI_{SEC}$ | *End-group fidelity* MALDI |
|---|---|---|---|---|---|---|
| a (2) | $\alpha$-BzO-P ($EG_{0.96}$-*co*-$GME_{0.04}$) | 5.3 | 4.8 | 3.9 | 1.04 | >99% |
| b (2) | $\alpha$-BzO P ($EG_{0.92}$-*co*-$GME_{0.08}$) | 5.2 | 5.3 | 4.5 | 1.05 | >99% |
| c (2) | $\alpha$-BzO P ($EG_{0.84}$-*co*-$GME_{0.16}$) | 6.9 | 4.9 | 3.7 | 1.05 | >99% |
| d (2) | $\alpha$-BzO P ($EG_{0.76}$-*co*-$GME_{0.24}$) | 6.0 | 5.2 | 3.8 | 1.06 | >99% |
| e (3) | $\alpha$-BzO P ($EG_{0.51}$-*co*-$GME_{0.49}$) | 4.2 | 3.8 | 2.4 | 1.09 | >99% |
| f (2) | $\alpha$-BzO P ($EG_{0.96}$-*co*-$GME_{0.04}$) | 10.9 | 8.5 | 7.5 | 1.08 | >99% |
| g (2) | $\alpha$-BzO P ($EG_{0.93}$-*co*-$GME_{0.07}$) | 9.0 | 8.5 | 7.3 | 1.05 | >99% |
| h (2) | $\alpha$-MeO-P ($EG_{0.97}$-*co*-$GME_{0.03}$) | 7.0 | 5.6 | 4.5 | 1.10 | >99% |
| i (2) | $\alpha$-DBzN-P ($EG_{0.97}$-*co*-$GME_{0.03}$) | 6.9 | 5.1 | 4.0 | 1.08 | >99% |
| j (4) | $\alpha$-DBzN-EtO-PGME | 4.2 | 4.0 | 2.7 | 1.05 | >99% |
| k (5) | $\alpha$-BzO-P ($EG_{0.85}$-*co*-$EGE_{0.15}$) | 6.7 | n.d. | 4.2 | 1.09 | n.d. |
| l (6) | $\alpha$-MeO- $PPEG_{0.88}$-*b*-$PGME_{0.22}$ | 3.6 | 3.7 | 2.8 | 1.05 | >99% |
| m (7) | $\alpha$-BzO-$\omega$-Ts-P($EG_{0.93}$-*co*-$GME_{0.07}$) | 11.1 | 8.8 | n.d. | n.d. | >99% |

$M_n$ = molecular weight (number average molar mass), PDI = polydispersity.

Immunogenicity

As can be seen from Table 3, entries a to e show copolymers of the present invention which consisting of ethylene oxide repeating units and glycidyl methyl ether repeating units with about the same molecular weight, i.e. with an $M_n$ of about 4 to 5 kg/mol. The amount of glycidyl methyl ether repeating units is increasing from a to e from 4 % to 49% (see Table 1, entries a to e). Figure 1 shows the ELISA test results of polymers c to e and of commercially available mPEG. It shows the function of the normalized absorption at a wavelength of 450 nm versus the log10 function of the polymer concentration in nanograms per ml, therefore illustrating the anti-PEG antibody interaction with the investigated polymer concentrations. The ELISA data shows that a strong influence of an increasing concentration of alkyl side chains can be observed (it is important to note that the x-axis has a logarithmic scale). With increasing amount of GME incorporated into the polyether structure, significantly higher polymer concentrations are necessary to observe interactions between the copolymer and the APA. Remarkably, at 49 mol% of GME, absolutely no interaction between APA and the copolymer is present. This means that the respective copolymer cannot be detected by APAs, i.e. it is not immunogenic.

Dispersity

As can be seen from Table 3, entries a to I, all polymers of the present invention have a very low dispersity, i.e. 1.10 or lower. For visualization, the SEC traces of these polymers are shown in Figures 2 to 8. Figure 8 shown in addition the SEC trace of mPEG. It can clearly be seen that the present invention provides polymers with a very narrow molecular weight distribution.

(In Abbildung 2 sollte die rote Linie gegen eine gepunktete Linie oder ähnliches ausgetauscht werden. Die rote Linie wird sonst in der Patentschrift nicht von der schwarzen durchgezogenen Linie unterscheidbar sein.

Preparation and purity of the polymers of the present invention

Figure 9 shows the $^1$H NMR spectrum of $\alpha$-BzO P($EG_{0.51}$-*co*-$GME_{0.49}$), i.e. the polymer of Example 3 (entry e of Tables 2 and 3). The product is as obtained from Example 3, without further purification. As can be seen, the present method does not only provide polymers of narrow molecular weight distribution, but also very pure polymers without the need for laborious purification steps. This is an important feature for the use in pharmaceutical applications.

End-group fidelity

As can be seen from Table 3, the end-group fidelity for all polymers of the present invention is nearly 100%. Figure 10 shows the MALDI TOF mass spectrum of $\alpha$-BzO P($EG_{0.51}$-*co*-$GME_{0.49}$), i.e. the polymer of Example 3 (entry e of Tables 2 and 3) with potassium and sodium cations. The only peaks visible here are the peaks of the product molecule. There are no peaks form macromolecules with a different end group. This shows that end-group fidelity of the polymers of the present invention is very high. In addition, the spectrum confirms the narrow molecular weight distribution.

Solubility

Figure 4 shows the cloud point measurement of $\alpha$-BzO-P(EG0.51-co-GME0.49), i.e. the polymer of Example 3 (entry e of Tables 2 and 3). The cloud point determines the temperature at which immiscibility in water / in aqueous solution is observed upon heating. As an example, for the copolymer with 49% GME, the cloud point can be observed at 95 °C, showing excellent water solubility of the Poly(GME-co-EO) copolymers. The homopolymer PEG shows a cloud point of ca. 100°C. All other copolymers of the present invention with 26-49% GME are in between PEG and this copolymer, showing cloud points of 96-100°C. For copolymers of 25 mol% GME incorporation and lower no cloud point was detected in water at temperatures ranging from 0 to 99 °C. Thus, GME copolymerization hardly affects water solubility, which is a feature of the similar structure of polyethylene glycol and methyl ethyl ether. This is an important feature of the polymers of the present invention as it shows that they can substitute PEG in aqueous systems without causing problems with phase separation or even precipitation.

Homopolymers

Figure 12 shows the MALDI-TOF spectrum of the homopolymer of Example 4. The spectrum shows not only that the polymer has a low dispersity, but also that it is free from impurities deriving different end groups. The fact that besides the peaks of the product only white noise is visible shows that the end-group fidelity is about 100 %. Glycidyl methyl ether homopolymer has so far not been produced with these specifications.

Comparison of dispersity of end group fidelity of the polymers of the present invention with that of mPEG

Figure 13 shows an overlay of MALDI TOF spectra of commercially available mPEG and a block copolymer of the present invention (polymer of Example 6 (entry I of Tables 2 and 3). As can be seen, a clear shift between the macroinitiator mPEG and entry I is observed, indicating a successful block copolymer synthesis. Absence of macroinitiator in the MALDI-ToF MS of entry I further proves quantitative block copolymer formation. This is further proven by a distance of the signals of 44 g mol-1.

Modification of end groups

Figure 14 shows the MALDI TOF spectrum of $\alpha$-BzO-ω-Ts-P(EG-co-GME), i.e. the polymer of Example 7 (see Tables 2 and 3, entry). In this polymer the $\omega$-end group OH has been modified to a tosyl group. As can be seen from the spectrum, the modified polymer has the same narrow molecular weight distribution and the same high end-group fidelity. This shows that the polymers of the present invention can be modified as known for PEG.

Crystallinity

Table 4 shows thermal properties of some of the polymers of Tables 2 and 3:

| Sample | $T_g$ | $T_m$ | $\Delta H_{PEG}$ | $X_{c,PEG}$ |
|---|---|---|---|---|
| | [°C] | [°C] | [J/g] | |
| a | -56 | 46 | 88.63 | 0.45 |
| b | -56 | 45 | 76.79 | 0.39 |
| c | -59 | 37 | 56.26 | 0.29 |
| f | -59 | 35 | 58.22 | 0.30 |
| q | -61 | 7 | 32.62 | 0.17 |
| k | -56 | 46 | 88.63 | 0.45 |
| l | -59 | 45 | 80.16 | 0.41 |

$T_g$ = Glass transition temperature; $T_m$ = Melting point; $\Delta H_{PEG}$ = Enthalpy of fusion; $X_c$ = Crystallinity (all measurements by DSC)l

As can be seen, crystallinity of the inventive polymers is low. This prevents for example accumulation of the polymers in the kidney or in the liver.

## Claims

**1.** Polyether polymers represented by the following formula [I]:

$$-(CH_2CHR\text{-}O\text{-})_m- \qquad [I]$$

wherein

- residues R are independently from each other selected from the group consisting of hydrogen, methoxymethyl, ethoxymethyl, n-propoxymethyl and iso-propoxymethyl;
- 1 to 100% of the residues R are methoxymethyl;
- up to 50% of the residues R may be selected from the group consisting of ethoxymethyl, n-propoxymethyl and iso-propoxymethyl;
- with the proviso that at least one residue R is hydrogen, if at least one residue R is selected from the group consisting of ethoxymethyl, n-propoxymethyl and iso-propoxymethyl; and
- m is in the range of from 10 to 1000,

**characterized in that**
the dispersity is 1.15 or less.

**2.** Polyether polymer according to claim 1, wherein 5 to 95% of the residues R are hydrogen and 5 to 95% of the residues R are independently from each other selected from the group consisting of methoxymethyl, ethoxymethyl, n-propoxymethyl and iso-propoxymethyl.

**3.** Polyether polymer according to any of the preceding claims, wherein the polyether is a poly(glycidyl methyl ether-co-ethylene oxide) copolymer.

**4.** Polyether polymer according to any of the preceding claims, wherein 30 to 70% of the residues R are hydrogen and the remaining residues R are methoxymethyl.

**5.** Polyether polymer according to any of the preceding claims, wherein the polymer is a random copolymer.

**6.** Polyether polymers according to any of the preceding claims represented by the following formula [II]:

$$X\text{-}(CH_2CHR\text{-}O\text{-})_mCH_2CHR\text{-}Y \qquad [II]$$

wherein X is selected from the group consisting of hydrogen, alkyl, hydroxy, sulfanyl, C1-C10-alkoxy, C1-C10-thioalkoxy, amino, amino-Cl-ClO-alkoxy, dibenzylamino-Cl-ClO-alkoxy, amide, N-heterocyclic carbenes and N-heterocyclic olefins;
Y is selected from the group consisting of hydrogen, hydroxy, alkoxy, $-CH_2-C(=O)-R$ wherein R is as defined above, -CHO, alkylcarbonyloxy, alkyoxycarbonyloxy, amine, C1-C10-alkylamine, carboxamido, azide, halogen, sulfanyl, thioalkoxy, N-succinimidyl carbonate and sulfonate;
X and/or Y may also be selected from the group consisting of low molecular weight drugs, nanocarriers, liposomal structures, peptides, polypeptides, proteins, glycoproteins, polynucleotide, polysaccharides, lipid structures, liposomes, surfaces and interfaces, which are bonded directly by a covalent bond or by a spacer to the polymer, m is in the range of from 9 to 999,

**characterized in that**
the dispersity is 1.15 or less.

**7.** Polyether polymer according to claim 6, wherein X is alkoxy and Y is hydroxy.

**8.** Polyether polymer according to any of claims 6 or 7, wherein end-group fidelity of the polymer in regard to group X and/or in regard to group Y is at least 95%.

**9.** Conjugate, comprising a polyether polymer of any of the preceding claims and a substrate.

**10.** Conjugate according to claim 9, wherein the substrate is selected from the groups consisting of low molecular weight drugs, nanocarriers, liposomal structures, peptides, polypeptides, proteins, glycoproteins, polynucleotides, polysaccharides, lipid structures, liposomes, surfaces and interfaces.

**11.** Process for the preparation of a polyether polymer according to any of claims 1 to 8, by anionic ring-opening copolymerization comprising the steps of:

- Providing an anion $An^-$,
- Adding at least one monomer of the formula 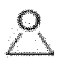 -R, where in R is as defined in any of claims 1 to 8,
- Allowing the polymerization to proceed at a temperature in the range of -10 to 90 °C

wherein the monomer comprises less than 1 wt % of epichlorohydrin.

**12.** Process according to claim 11, wherein the step of adding at least one monomer and the step of allowing the polymerization to proceed are repeated at least one time, whereby at least one different monomer is used than the first time.

**13.** Process according to any of claims 11 or 12, wherein the counter ion to the $An^-$ anion is selected from the group consisting of $Na^+$, $K^+$ and $Cs^+$.

**14.** Use of the polyether polymers according to any one of claims 1 to 8 for the preparation of a conjugate of the polymers with a bioactive compound.

**15.** Use according to claim 14 for the preparation of conjugated lipids for use in vaccines, in particular based on lipid nanoparticles, wherein these nanoparticles are preferably nanoparticles, as used against COVID-19.

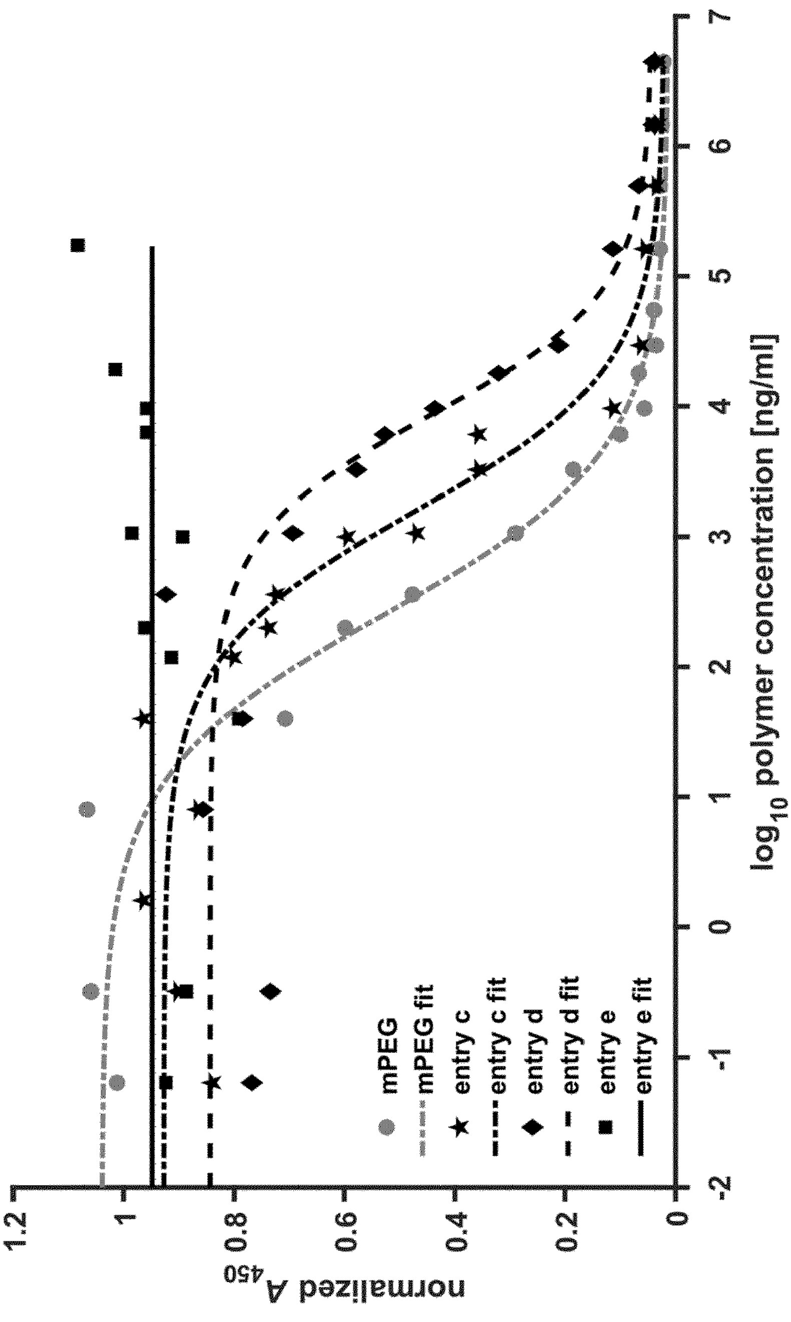
mPEG
mPEG fit
entry c
entry c fit
entry d
entry d fit
entry e
entry e fit
normalized $A_{450}$
$log_{10}$ polymer concentration [ng/ml]
1.2
1
0.8
0.6
0.4
0.2
0
-2
-1
0
1
2
3
4
5
6
7

Figure 1

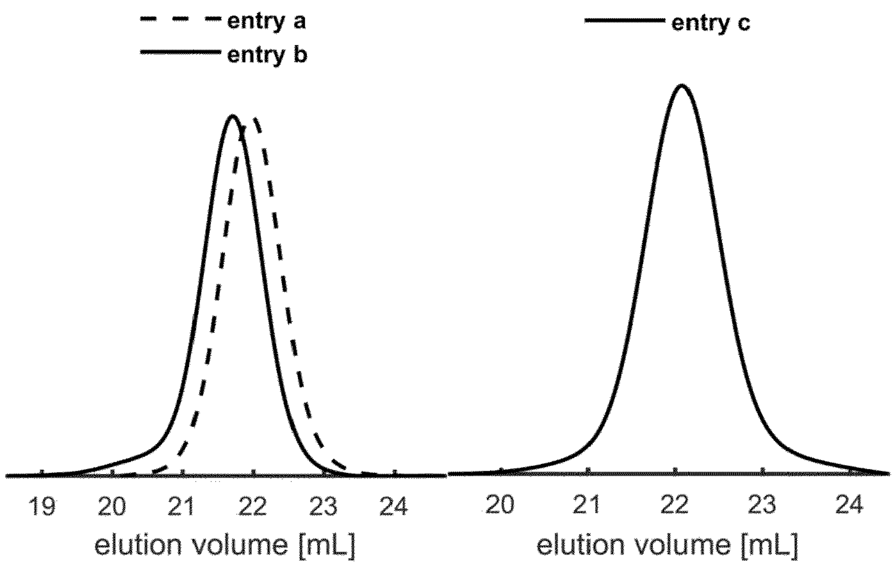


Figure 2

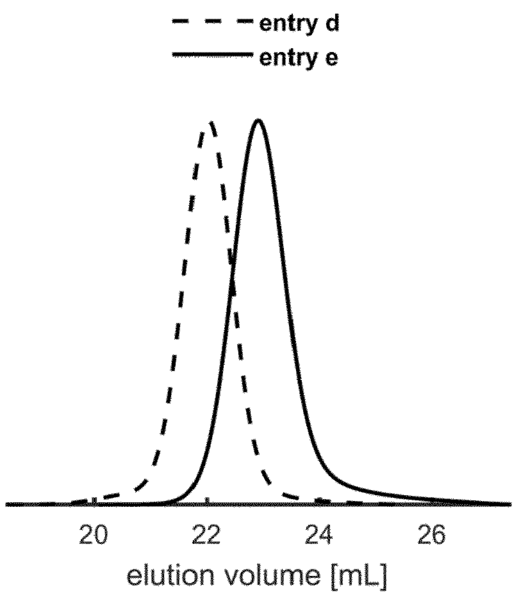


Figure 3

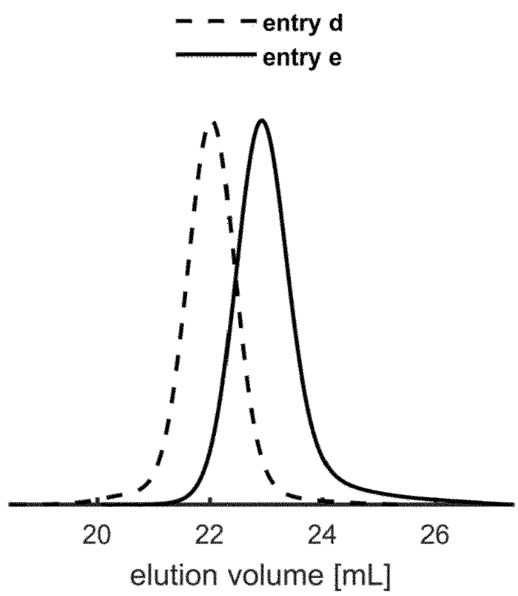


Figure 4

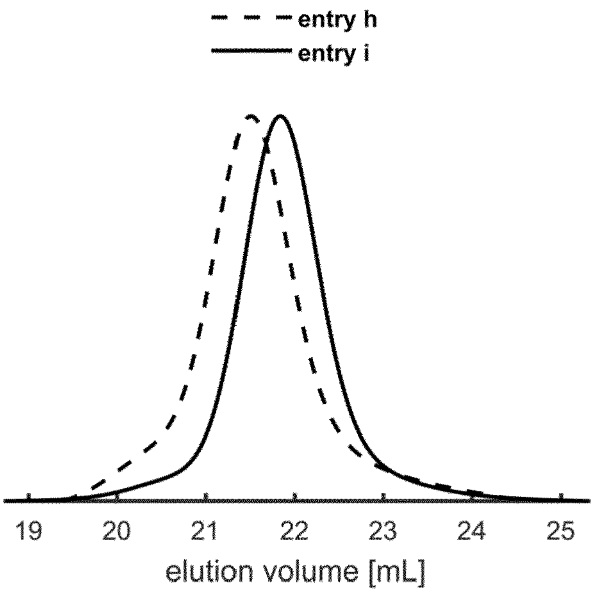


Figure 5

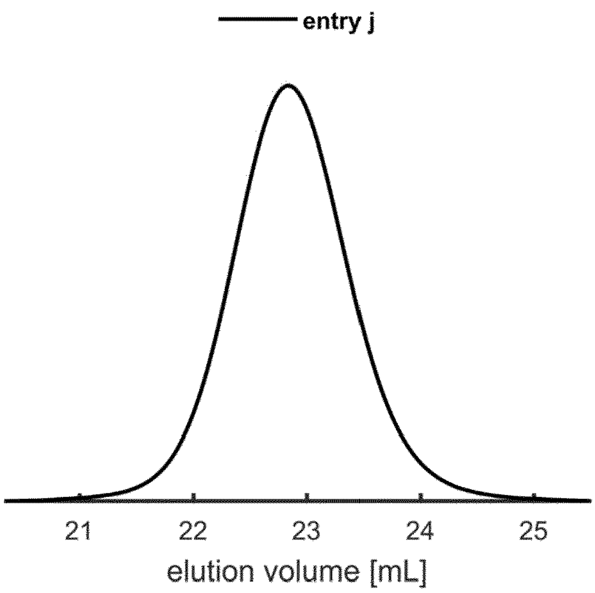


Figure 6

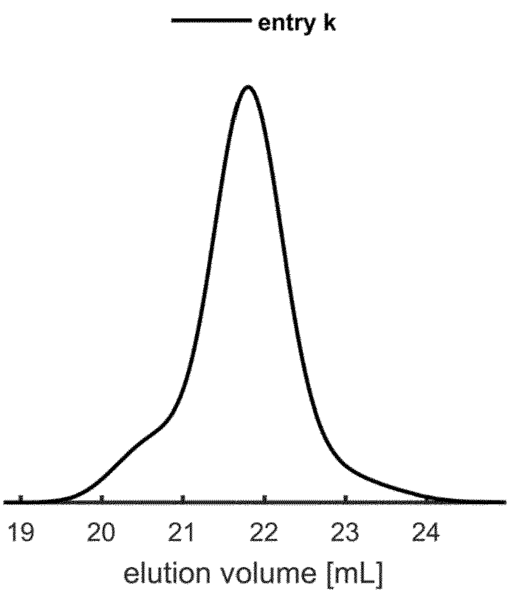


Figure 7

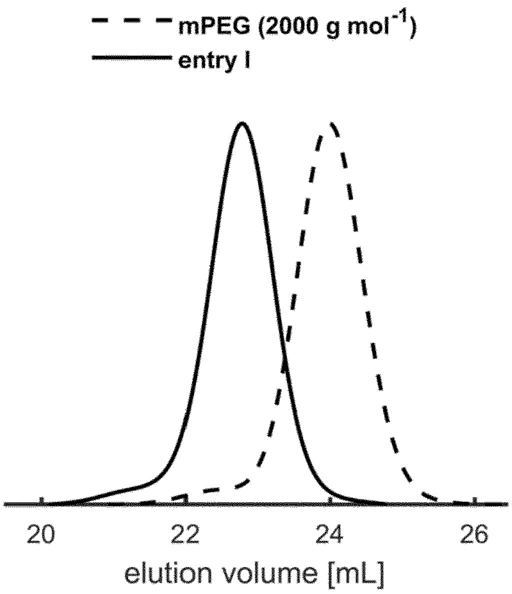


Figure 8

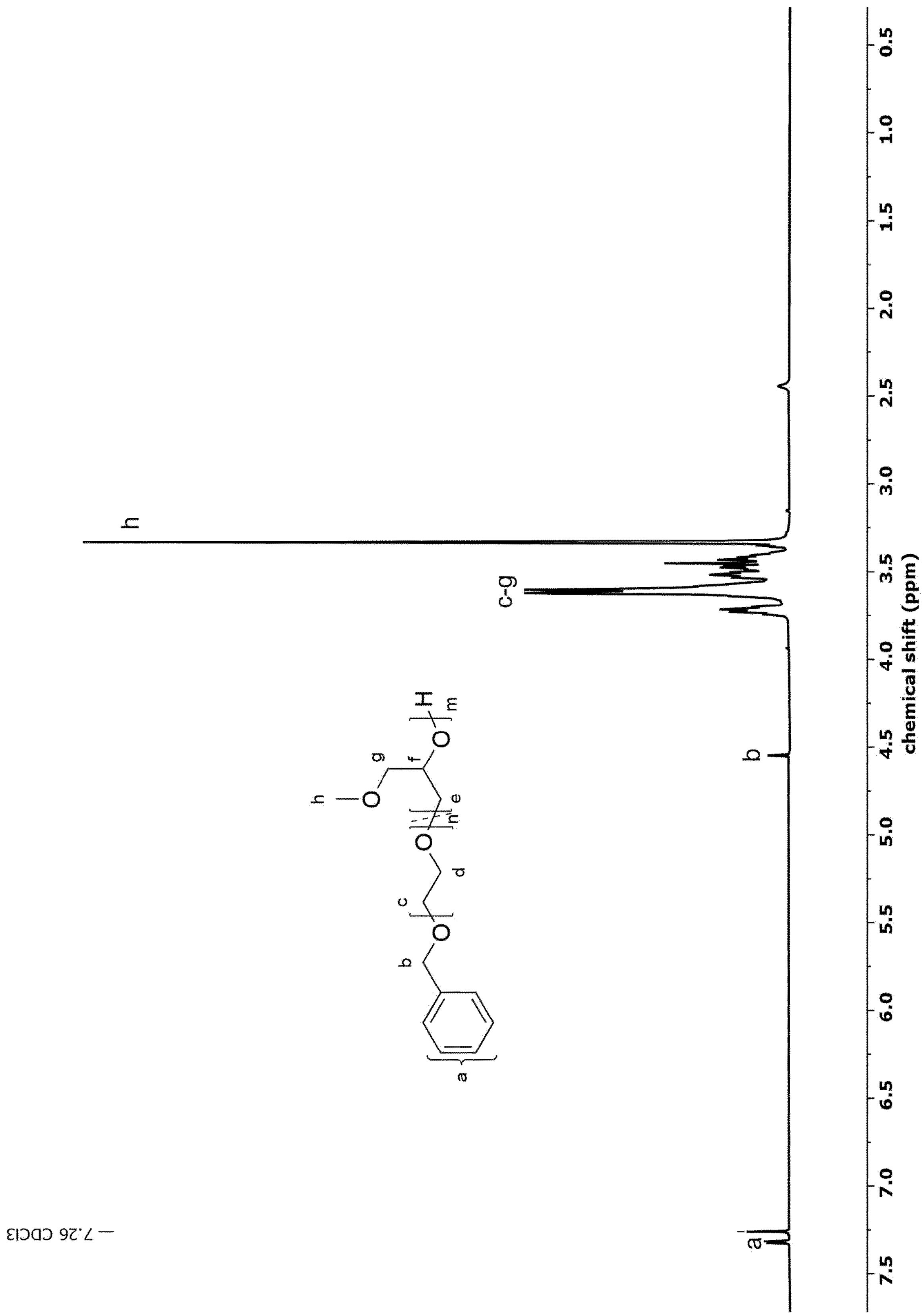
7.26 CDCl3
h
c-g
b
a
0.5
1.0
1.5
2.0
2.5
3.0
3.5
4.0
4.5
5.0
5.5
6.0
6.5
7.0
7.5
chemical shift (ppm)

Figure 9

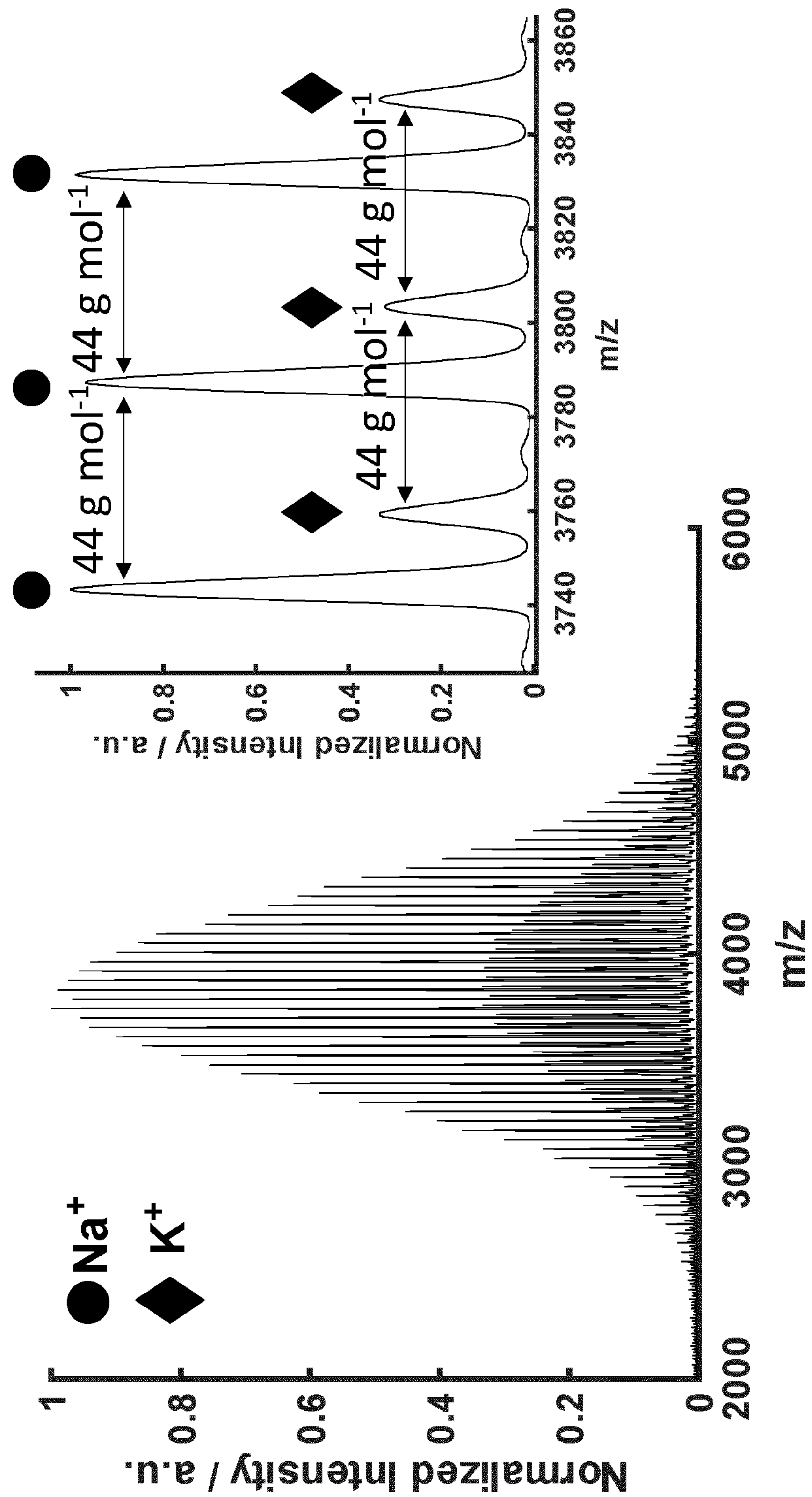

Na+
K+
Normalized Intensity / a.u.
m/z
44 g mol-1
44 g mol-1
44 g mol-1
44 g mol-1


Figure 10

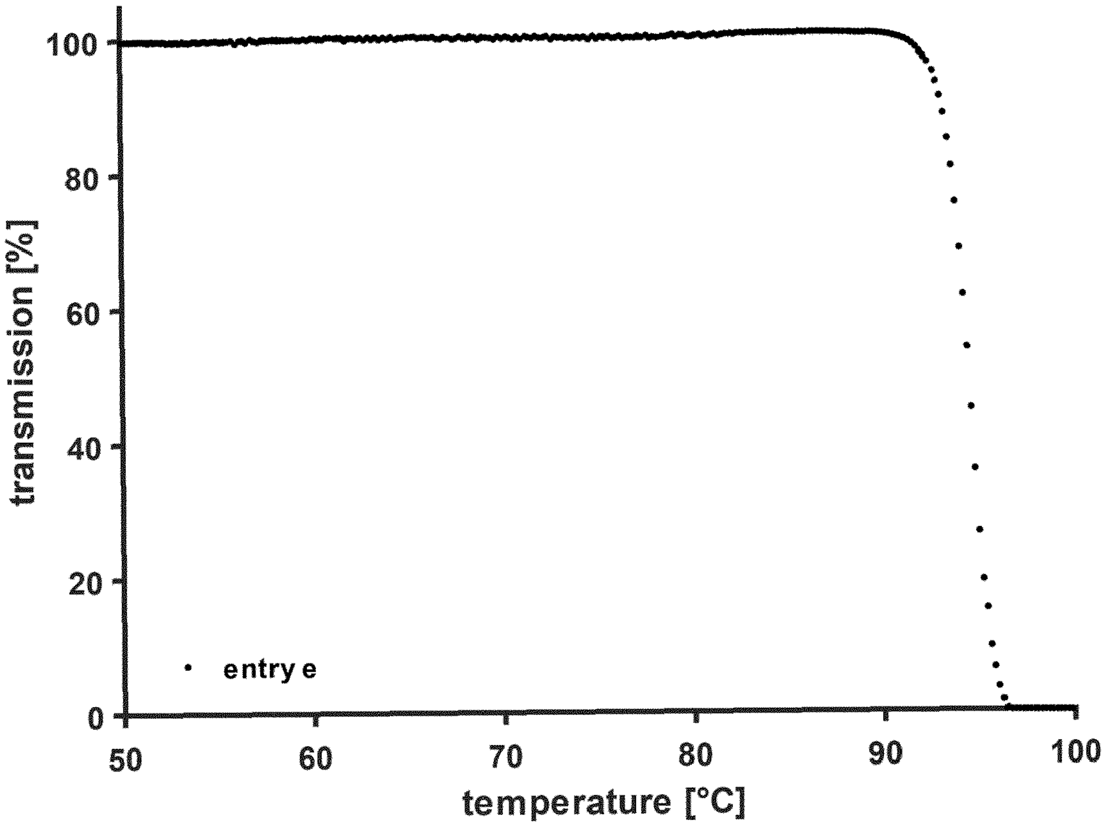
100
80
60
40
20
0
transmission [%]
entry e
50
60
70
80
90
100
temperature [°C]

Figure 11

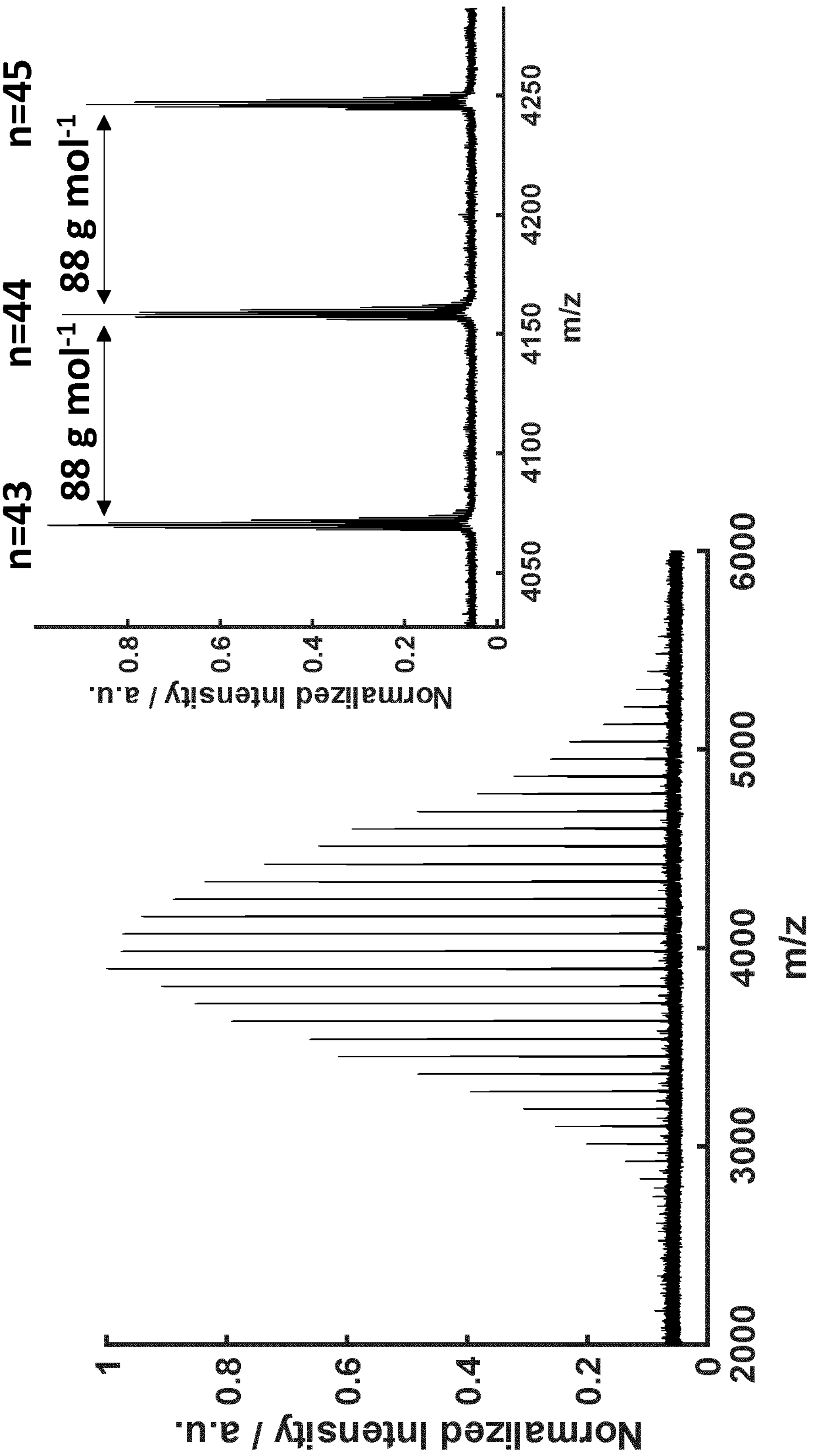
n=43
n=44
n=45
88 g mol-1
88 g mol-1
Normalized Intensity / a.u.
m/z
4050
4100
4150
4200
4250
0
0.2
0.4
0.6
0.8
1
2000
3000
4000
5000
6000

Figure 12

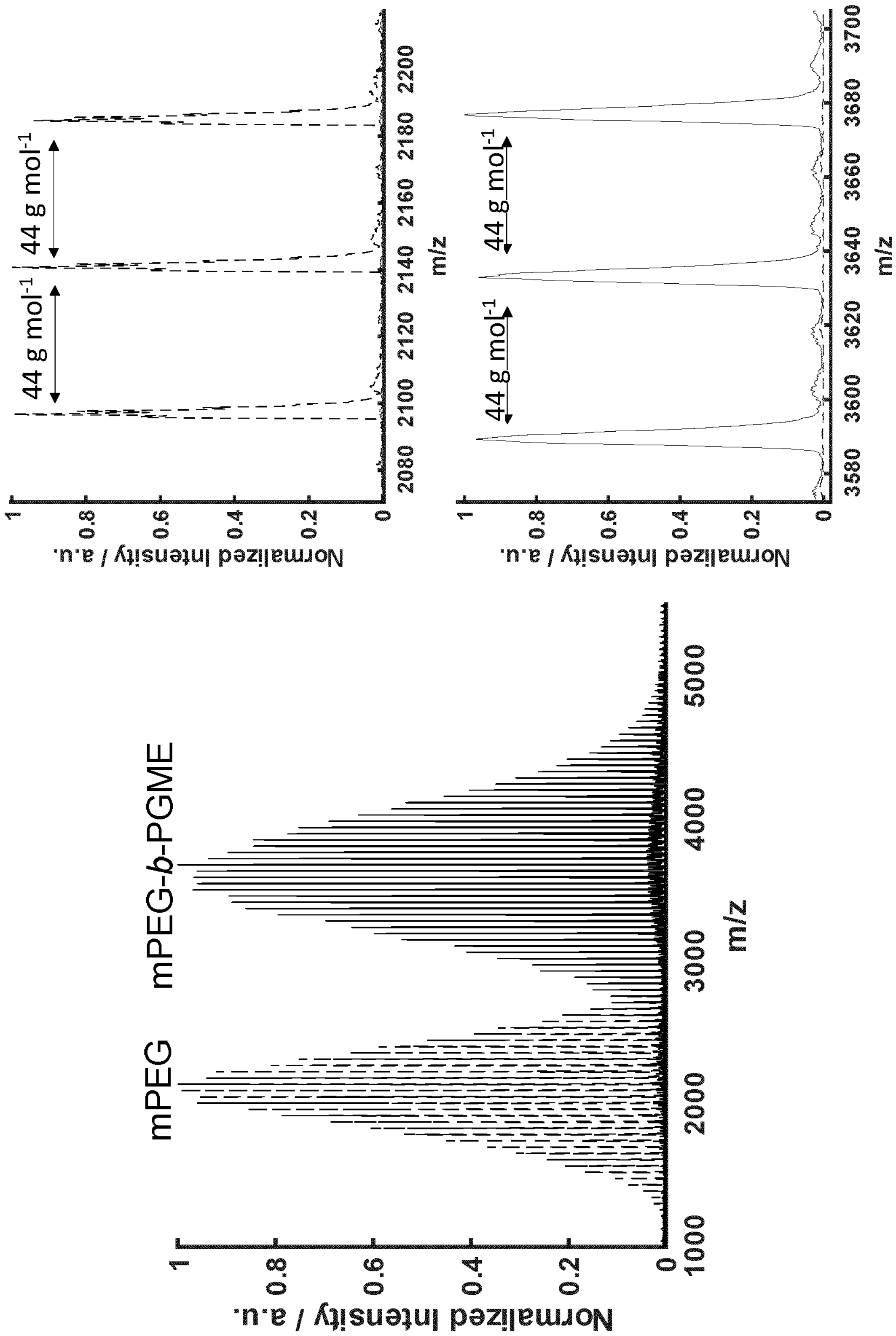
mPEG
mPEG-b-PGME
Normalized Intensity / a.u.
m/z
44 g mol-1
44 g mol-1
Normalized Intensity / a.u.
m/z
44 g mol-1
44 g mol-1
Normalized Intensity / a.u.
m/z

Figure 13

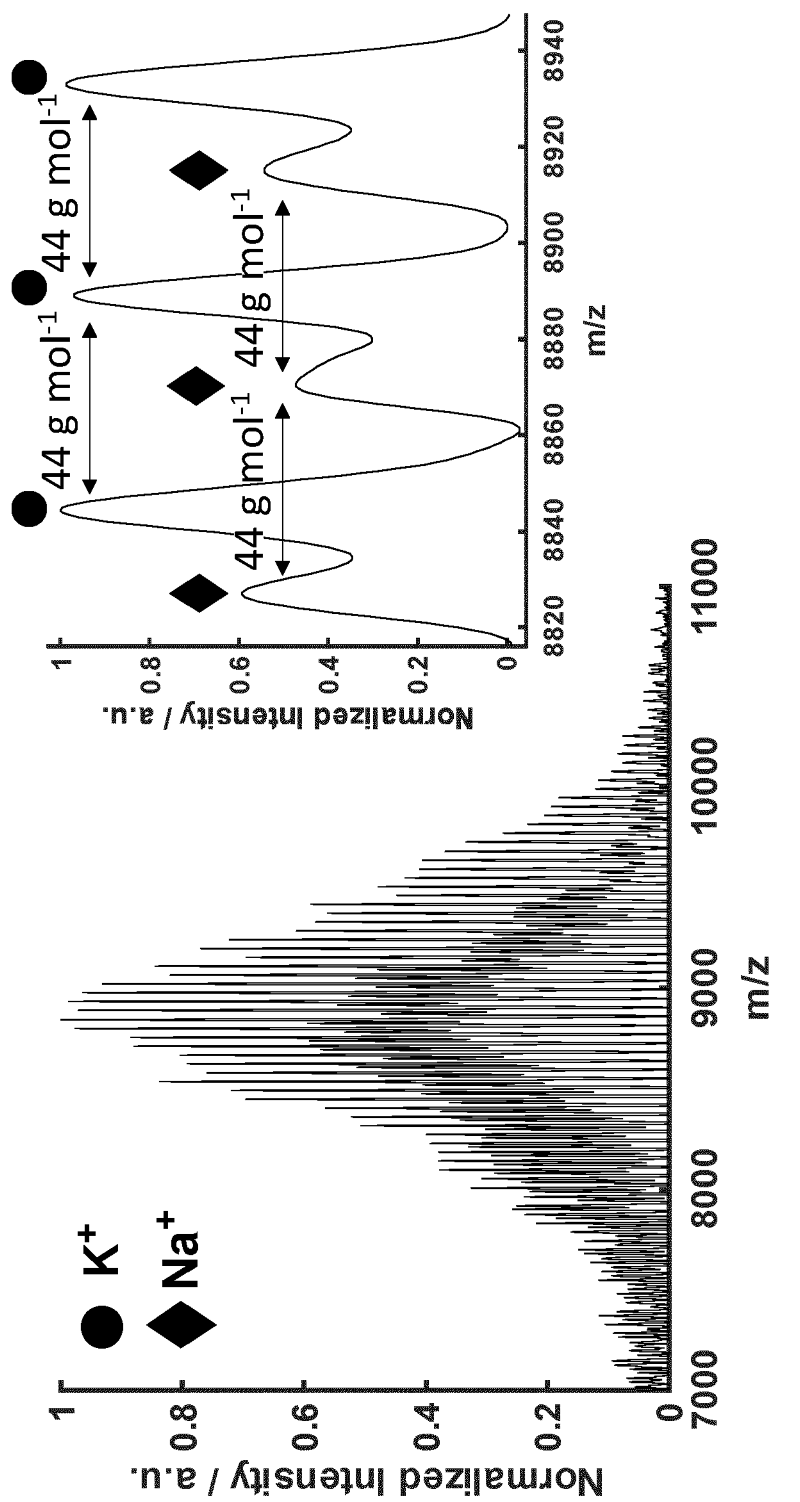
K+
Na+
Normalized Intensity / a.u.
m/z
44 g mol-1
44 g mol-1
44 g mol-1
44 g mol-1
7000
8000
9000
10000
11000
0
0.2
0.4
0.6
0.8
1
8820
8840
8860
8880
8900
8920
8940

Figure 14

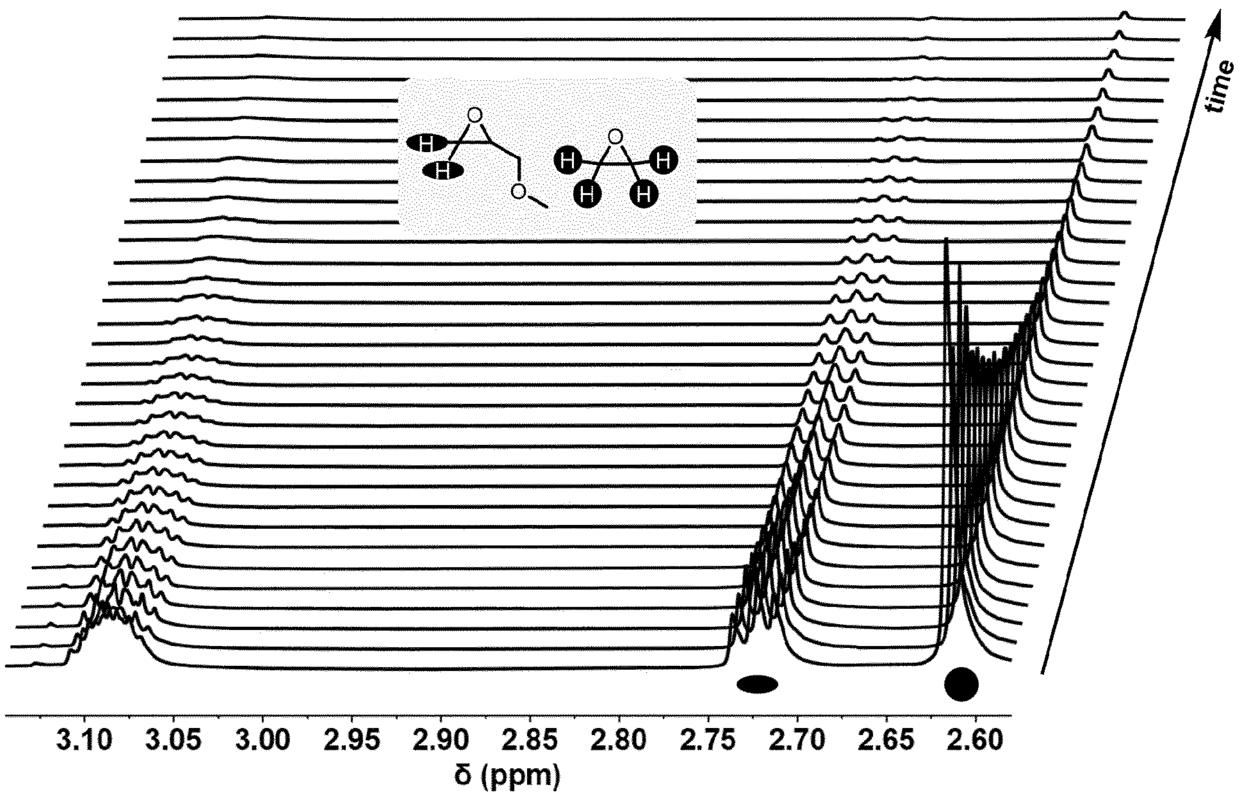


Figure 15

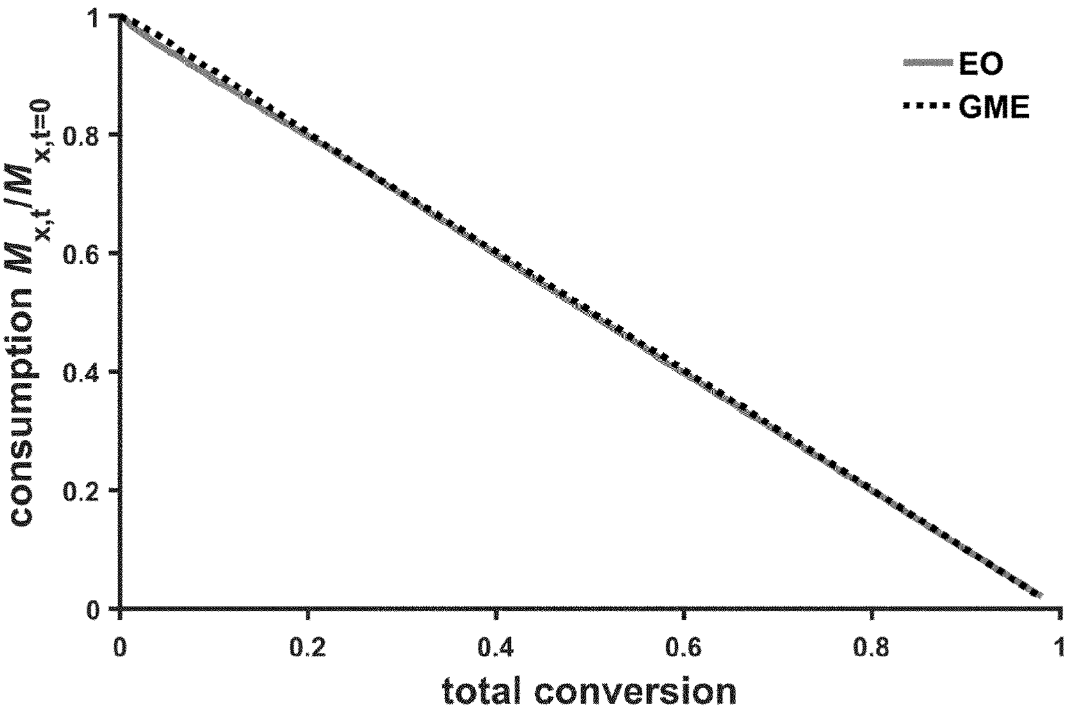


Figure 16

Europäisches Patentamt
European Patent Office
Office européen des brevets

# EUROPEAN SEARCH REPORT

Application Number
EP 21 17 3944

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | FELLIN C.R. ET AL: "Tunable temperature and shear-responsive hydrogels based on poly(alkyl glycidyl ether)", POLYMER INTERNATIONAL, vol. 68, 30 October 2018 (2018-10-30), pages 1238-1246, XP002804686, * polymers 1,3,8,9 and 10; table 2 * * table 1 * * scheme 1 * * scheme 2 * * Experimental -materials; page 1239 * | 1,2,6,8 | INV. C08G65/22 A61K47/60 C08G65/333 |
| X | JUNG H. ET AL: "Regulating dynamics of polyether-based triblock copolymer hydrogels by end block hydrophobicity.", MACROMOLECULES, vol. 53, 20 October 2020 (2020-10-20), pages 10339-10348, XP002804687, * table 1 * * figure 1 * * Experimental section; page 10340 * | 1,2,6,8 | |
| | | | TECHNICAL FIELDS SEARCHED (IPC) |
| X | LABBÉ A. ET AL: "Controlled polymerisation of glycidyl methyl ether initiated by onium/triisobutylaluminium and an investigation of the polymer LCST.", MACROMOL.SYMP., vol. 249-250, 2007, pages 392-397, XP002804688, * table 1 * | 1 | C08G A61K |

-/--

2

The present search report has been drawn up for all claims

| Place of search | Date of completion of the search | Examiner |
|---|---|---|
| The Hague | 5 November 2021 | O'Sullivan, Timothy |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

page 1 of 2

Europäisches Patentamt
European Patent Office
Office européen des brevets

# EUROPEAN SEARCH REPORT

Application Number
EP 21 17 3944

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | EP 2 022 808 A1 (DOW CORNING TORAY CO LTD [JP]) 11 February 2009 (2009-02-11)<br>* paragraph [0080] *<br>* examples 1-4 *<br>----- | 1-3, 11-13 | |
| A | US 2005/113560 A1 (SUGAWARA SHUICHI [JP]) 26 May 2005 (2005-05-26)<br>* claims 1,15, *<br>----- | 1-15 | |
| A,D | FREY ET AL.: "A Challenging Comonomer Pair: ''Copolymerization of Ethylene Oxide and Glycidyl Methyl Ether to Thermoresponsive Polyethers", MACROMOLECULES, vol. 47, 2014, pages 5492-5500, XP002804689,<br>* table 1 *<br>----- | 1-15 | |
| A | US 2020/407479 A1 (VERKOYEN PATRICK [DE] ET AL) 31 December 2020 (2020-12-31)<br>* claim 1 *<br>* table 2 *<br>----- | 1-15 | TECHNICAL FIELDS SEARCHED (IPC) |

The present search report has been drawn up for all claims

| Place of search | Date of completion of the search | Examiner |
|---|---|---|
| The Hague | 5 November 2021 | O'Sullivan, Timothy |

2

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

# ANNEX TO THE EUROPEAN SEARCH REPORT ON EUROPEAN PATENT APPLICATION NO. EP 21 17 3944

This annex lists the patent family members relating to the patent documents cited in the above-mentioned European search report.
The members are as contained in the European Patent Office EDP file on
The European Patent Office is in no way liable for these particulars which are merely given for the purpose of information.

05-11-2021

| Patent document cited in search report | Publication date | Patent family member(s) | Publication date |
|---|---|---|---|
| EP 2022808 A1 | 11-02-2009 | CN 101448873 A | 03-06-2009 |
| | | EP 2022808 A1 | 11-02-2009 |
| | | JP 5525728 B2 | 18-06-2014 |
| | | JP WO2007135770 A1 | 01-10-2009 |
| | | US 2010022732 A1 | 28-01-2010 |
| | | WO 2007135770 A1 | 29-11-2007 |
| US 2005113560 A1 | 26-05-2005 | AU 2003281208 A1 | 23-01-2004 |
| | | JP 4925581 B2 | 25-04-2012 |
| | | JP WO2004004794 A1 | 04-11-2005 |
| | | US 2005113560 A1 | 26-05-2005 |
| | | WO 2004004794 A1 | 15-01-2004 |
| US 2020407479 A1 | 31-12-2020 | DE 102018107464 A1 | 02-10-2019 |
| | | EP 3774990 A1 | 17-02-2021 |
| | | US 2020407479 A1 | 31-12-2020 |
| | | WO 2019185719 A1 | 03-10-2019 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 8129330 B2
- US 20190015520 A1


### Non-patent literature cited in the description


- **YAROVSKY et al.** Quantitative design rules for protein-resistant surface coatings using machine learning. *Scientific Rep.,* 2019, vol. 9, 265
- **LAI et al.** Structure of an anti-PEG antibody reveals an open ring that captures highly flexible PEG polymers. *Commun Chem,* 2020, vol. 3, 124
- **SHERMAN et al.** Role of the methoxy group in immune responses to mPEG-protein conjugates. *Bioconjug Chem,* 2012, vol. 23 (3), 485-499
- **SHERMAN et al.** Selectivity of binding of PEGs and PEG-like oligomers to anti-PEG antibodies induced by methoxyPEG-proteins. *Mol Immunol,* 2014, vol. 57 (2), 236-246
- **LIU et al.** Antibodies Predict Pegasparagase Allergic Reactions and Failure of Rechallenge. *J. Clin. Oncol.,* 2019, vol. 37, 2051
- **ABU LILA et al.** Use of polyglycerol (PG), instead of polyethylene glycol (PEG), prevents induction of the accelerated blood clearance phenomenon against long-circulating liposomes upon repeated administration. *International Journal of Pharmaceutics,* 2013, vol. 456, 235-242
- **BENDELE, A. ; SEELY, J. ; RICHEY, C. ; SENNELLO, G. ; SHOPP, G.** Short Communication: Renal Tubular Vacuolation in Animals Treated with Polyethylene-Glycol-Conjugated Proteins. *Toxicol. Sei.,* 1998, vol. 42, 152-157
- **FREY et al.** A Challenging Comonomer Pair: ''Copolymerization of Ethylene Oxide and Glycidyl Methyl Ether to Thermoresponsive Polyethers''. *Macromolecules,* 2014, vol. 47, 5492-5500
- **FREY et al.** *Biomacromolecules,* 2014, vol. 15, 1935-1954